# EUROPEAN PATENT APPLICATION

(11) **EP 2 674 497 A1**
(43) Date of publication of application: **18.12.2013**
(21) Application number: 12004422.7
(22) Date of filing: 12.06.2012
(51) Int. Cl.: C12N 15/85

(54) **A light regulated transgene expression system**

(71) Applicant: Baden-Württemberg Stiftung gGmbH, 70191 Stuttgart (DE)
(72) Inventor: Weber, Wilfried, Prof. Dr., 79100 Freiburg (DE); Zubriggen, Matias, Dr., 79114 Freiburg (DE); Müller, Konrad, 79312 Emmendingen (DE); Kämpf, Michael, dR., 84503 Altötting (DE); Nagy, Ference, Prof. Dr., 79100 Freiburg (DE)
(74) Representative: Müller-Boré & Partner Patentanwälte

(57) **Abstract**

The present invention relates to a system for the light-regulated expression of a transgene in a mammalian cell which can repeatedly and reversibly be activated and inactivated by illumination with light, based on the use of a split transcription factor that is present in the nucleus. The present invention further relates to an isolated nucleic acid molecule encoding said split transcription factor, a vector comprising said nucleic acid, a host cell comprising said system, isolated nucleic acid or vector, and a method for the light-regulated expression of a transgene in a cell, said method using the system of the present invention. Furthermore, the present invention relates to a kit for performing said method, and uses of the above.

## Description

The present invention relates to a system for the light-regulated expression of a transgene in a mammalian cell which can repeatedly and reversibly be activated and inactivated by illumination with light, based on the use of a split transcription factor that is present in the nucleus. The present invention further relates to an isolated nucleic acid molecule encoding said split transcription factor, a vector comprising said nucleic acid, a host cell comprising said system, isolated nucleic acid or vector, and a method for the light-regulated expression of a transgene in a cell, said method using the system of the present invention. Furthermore, the present invention relates to a kit for performing said method, and uses of the above.

Inducible expression systems to control transgene activity represent a cornerstone technology in recombinant cell technology and synthetic biology. In particular, such systems are of great value in the production of proteins that cannot be produced by classical batch-fermentation at optimal levels. The recent emergence of chemically inducible expression systems has paved the way for significant improvements in the expression of certain proteins, such as proteins that are of particular interest as biopharmaceuticals. It also enabled the conditional engineering of cell metabolism for the best production performance.

The most widely used inducible expression systems rely on adjustable promoters that can be triggered by small-molecule inducers such as antibiotics, hormones, immunosuppressants, volatile aldehydes or quorum-sensing messengers. Moreover, findings in the field of light-sensing proteins have led to the emergence of expression system using light as an inducer of gene expression. In line with these findings, a number of optogenetic gene expression systems have been developed over the last years. Besides systems using caged compounds, these comprise phytochrome- and cryptochrome-based systems developed in yeast. For light inducible gene expression in mammalian cells, blue-light responsive systems have been reported that are based on light-oxygen-voltage (LOV) domains or on the channel protein melanopsin.

However, gene expression system known in the art all suffer from several inherent drawbacks.

In particular, classical gene expression systems that are not inducible and rely on continuous production processes wherein the freshly produced target protein is continuously withdrawn from the cells and immediately purified are highly complex, expensive, and are prone to cell drifting and contamination due to prolonged production campaigns over up to several weeks.

Further, the need for small-molecule inducers in chemically inducible expression system entails several inherent drawbacks that limit the advancement of recombinant cell technology. In particular, most of the small molecules used as inducers for expression of the product are either not licensed for therapeutic application, incompatible with downstream processing regulations or render the process non-profitable. Moreover, pharmacologically active inducers might evoke undesired side effects on the target cells and impose additional regulatory hurdles for the production of biopharmaceuticals. Furthermore, these inducers diffuse freely and, therefore, do not allow a spatial control of gene expression.

In contrast to chemical inducers, light at a cell-compatible wavelength is nontoxic, non-invasive, easy to obtain and enables gene expression with spatiotemporal resolution. However, light-inducible expression systems known in the art such as systems using caged compounds require addition of the said caged compound and UV-radiation. More importantly, said systems are not reversible, i.e. expression can be turned constitutively on, but cannot be turned off at a later stage.

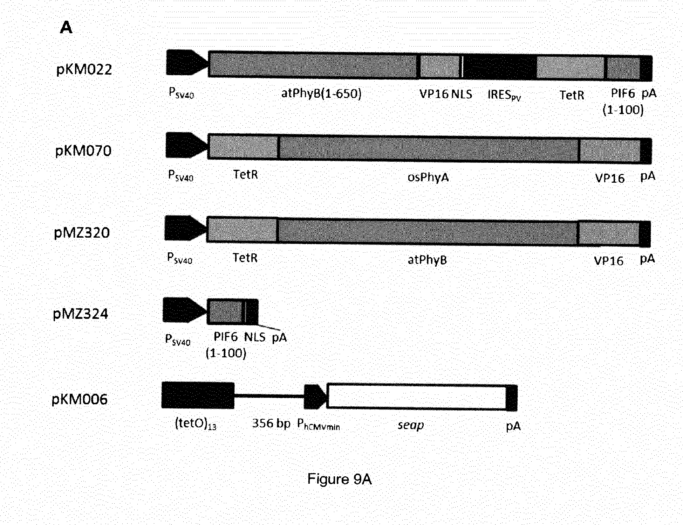

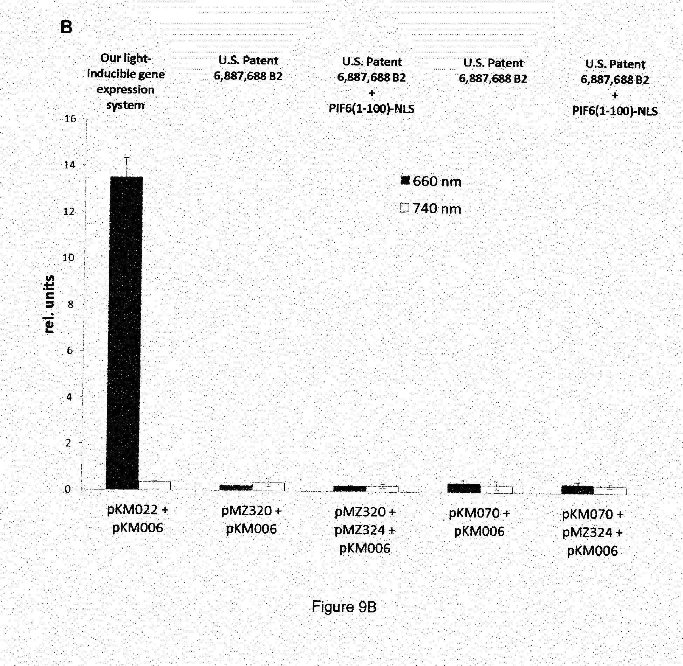

As an example of a phytochrome-based system known in the art, US-Patent 6,887,688 B2 *inter alia* discloses a system wherein heterologous phytochromes are used to translocate transactivators into the nucleus of a cell. In particular, fusion proteins of a DNA-binding protein, a phytochrome, e.g. Phytochrome A or B, and a transactivator are envisaged which should translocate to the nucleus upon light activation and activate a transgene of interest via the transactivator. However, cells carrying respective constructs do not show any light-inducible transgene expression (cf. Example 7 and Fig. 9, of the present application). This non-functionality is in line with recent literature findings indicating that additional plant factors are necessary to mediate light-inducible nuclear transport of phytochromes (Pfeiffer, A., M. K. Nagel, et al. (2012), "Interaction with plant transcription factors can mediate nuclear import of phytochrome B." Proc Natl Acad Sci U S A 109(15): 5892-5897; Pfeiffer, A., T. Kunkel, et al. (2009), "A cell-free system for light-dependent nuclear import of phytochrome." Plant J 57(4): 680-689).

As a further example of a phytochrome-based system known in the art, Sorokina *et al.* (Sorokina, O. et al. (2009), "A switchable light-input, light-output system modelled and constructed in yeast", Journal of Biological Engineering 3(15)) describes *inter alia* a light-responsive gene promoter system in yeast which is based on the use of a split transcription factor consisting of a construct comprising Phytochrome B (PhyB) and the DNA binding domain GBD and a construct comprising phytochrome interacting factor 3 (PIF3) and the translational transactivator GAD. Upon activation of PhyB by light, PhyB binds to PIF3 and the transcription factor is reconstituted. However, this system has been realized in yeast and not in mammalian cells. In this context, it is worth to note that there has been a long-standing technical prejudice in the field against the use of phytochrome-based expression systems such as the one described in Sorokina *et al.* in mammalian cells. In particular, it was believed that biliverdin reductase, an enzyme that is present in all mammalian cells, would catabolize and degrade Phycocyanobilin (PCB), *i.e*. the phytochrome's chromophore, thus rendering a respective expression system useless. This believe is supported by Terry *et al.* (Terry, M. J. *et al.* (1993), "Inactivation of Phytochrome- and Phycobiliprotein-Chromophore Precursors by Rat Liver Biliverdin Reductase",

The Journal of Biological Chemistry 268(35): 26099-26106), showing that PCB is catabolized by Biliverdin reductase and can no longer be used by phytochromes. Moreover, Lagarias *et al.* (Lagarias, D. M. et al. (1997), "Regulation of Photomorphogenesis by Expression of Mammalian Biliverdin Reductase in Transgenic Arabidopsis Plants", The Plant Cell 9: 675*688) shows the use of mammalian biliverdin reductase for the generation of a PCB-and, thus, phytochrome-deficient phenotype.

In summary, no light-regulated gene expression system for mammalian cells has been reported that could provide (i) the ability to be reversibly turned on and off multiple times in a controlled manner, (ii) fast kinetics of induction and inhibition of induction, and (iii) high expression rates at minimal background expression. The availability of such a system would be highly beneficial to recombinant cell technology and the production of proteins, in particular biopharmaceutical proteins.

Therefore, the technical problem underlying the present invention is to provide a light-regulated system for the expression of a transgene in a mammalian cell which (i) can be reversibly turned on and off multiple times in a controlled manner, (ii) displays fast kinetics of induction and inhibition of induction, and (iii) displays high expression rates at minimal background expression.

The solution to the above technical problem is achieved by the embodiments characterized in the claims.

The features of an optimal inducible gene expression system have been described by various authors. The light-regulated gene expression system of the present invention, meets all of the called for criteria.

In particular, the gene expression system of the present invention is based on the use of a split transcription factor for control of the transcription of a gene of interest. This split transcription factor comprises two fusion proteins, the first fusion protein comprising a photoreceptor protein, a transcriptional activator or a DNA-binding protein and a nuclear localization sequence (NLS), and the second fusion protein comprising a DNA-binding protein or a transcriptional activator and a factor that interacts with the photoreceptor protein in a light-dependent manner, wherein in case the first fusion protein comprises a transcriptional activator, the second fusion protein comprises a DNA-binding protein, and in case the first fusion protein comprises a DNA-binding protein, the second fusion protein comprises a transcriptional activator. Further, the system of the present invention utilizes a nucleic acid comprising at least one copy of an operator sequence which can be bound by said DNA-binding protein, a promoter that is responsive to activation by said transcriptional activator, and the coding sequence of the gene of interest under control of said promoter.

In the inactivated state, the first or second fusion protein is bound to said nucleic acid via binding of the DNA-binding protein to the operator sequence, providing a tether for said factor that interacts with the photoreceptor protein in a light-dependent manner or the DNA-binding protein interacts with the photochrome. Further, the first fusion protein is immediately present in the nucleus of the cell due to the NLS, thus providing very fast kinetics of induction. Upon absorption of light of a particular wavelength, the photoreceptor protein undergoes a conformational change that provides the ability to bind the above factor. Accordingly, heterodimerization of the split transcription factor is triggered, bringing the transcriptional activator in close proximity of the promoter which leads to the immediate initiation of transcription of the gene of interest. Absorption of light of another particular wavelength again triggers a conformational change of the photoreceptor back to its inactivated form, leading to dissociation of the split transcription factor and the immediate termination of transcription of the gene of interest. These processes can be repeatedly triggered by illumination with light having the respective wavelengths.

Due to this unique design and active principle, the system of the present invention provides a number of advantageous properties as compared to known expression systems. In particular, said system allows the expression of any gene of interest in a light-regulated manner, thus eliminating the need for any potentially harmful chemical inducers. Expression of the gene of interest can be turned on and off repeatedly for any number of times by illumination with light having the respective wavelengths. Due to the immediate presence in the nucleus and the very fast conformational changes of the light receptor protein in response to absorption of light, induction of expression and inhibition of expression display very rapid kinetics, in contrast to the slow kinetics of chemical induction which is e.g. dependent on diffusion of the inducer. In this context, it is worth to note that the conformational changes of the photoreceptor protein that lead to the establishment or termination of the interaction between said protein and the interacting factor can be triggered upon absorption of a single photon of the respective wavelength. Further, selection of the appropriate photoreceptor protein allows the use of light having particular wavelengths, e.g. of red/far red light which is less harmful to cells or tissues and can penetrate tissues more deeply.

Further, the expression system of the present invention provides a very low background expression while showing high expression levels upon induction. Moreover, expression levels can be precisely regulated via the amount of light used for induction and/or the amount of chromophore that is available for the photoreceptor protein. The expression system of the present invention is extremely sensitive and illumination with light for 25 min with 0.5 µmol m⁻²s⁻¹ can be sufficient for full activation. The system of the present invention can be used in any kind of mammalian cells. Thus, in case a respective red light-sensitive photoreceptor is used, the present invention provides *inter alia* the first red light-inducible expression system in mammalian cells. Furthermore, the system of the present invention allows the precise spatial control of gene expression by using e.g. photomasks which provide illumination and induction of gene expression of particular cells or tissues, while leaving other cells or tissues in the dark and, thus, gene expression inactivated.

Furthermore, the gene expression system of the present invention does not suffer from dark reversion, *i.e*. a decay or complete loss of gene expression in the dark. In particular, once activated, the gene expression system of the present invention is not dependent on activating light and gene expression remains active until the system is specifically deactivated by illumination with light of the respective wavelength. Further, the system of the present invention allows production of the transgene of interest in batch systems, wherein cells can be cultured to a desired cell density, the gene expression is activated for an optimal yield and the produced protein is then immediately harvested.

The genetic design of the system is compact and easy to handle, e.g. consisting of only two plasmids, one comprising the nucleic acids (a) and (b) as defined hereinbelow and one comprising nucleic acid (c) as defined hereinbelow. In certain applications of the system of the present invention, handling of the system of the present invention is further simplified as compared to other systems that use endogenous chromophores, since the system can be stored at daylight and gene expression can be triggered by the addition of the photoreceptor protein's chromophore, provided no chromophore is present beforehand. Moreover, in addition to the external addition of the chromophore, the system of the present invention allows also the endogenous production of the chromophore by co-expression of respective enzymes.

In summary, a synthetic light-regulated gene expression system for mammalian cells was engineered that is based on a split transcription factor. Light-induced reconstitution of the transcription factor triggers high expression levels that are rapidly and reversibly induced. The replacement of chemical induction by light-induced transgene expression retains the advantages of chemically inducible expression systems, while simultaneously eliminating the problems arising from the presence of these chemicals in the production system. The light-regulated protein expression system of the present invention can be implemented using conventional batch-type bioreactors with minimal modifications and at low cost. It has been demonstrated that said system is tunable and enables spatial control of gene expression, thus underlining its wide applicability to manipulate a multitude of biological processes.

In particular, in a first aspect the present invention relates to a system for the light-regulated expression of a transgene in a mammalian cell, comprising:
(a) a nucleic acid comprising the coding sequence of a first fusion protein, the first fusion protein comprising:
   (i) a photoreceptor protein;
   (ii) a transcriptional activator or a DNA-binding protein; and
   (iii) a nuclear localization sequence (NLS); and
(b) a nucleic acid comprising the coding sequence of a second fusion protein, the second fusion protein comprising:
   (i) a DNA-binding protein or a transcriptional activator; and
   (ii) a factor that interacts with the photoreceptor protein in a light-dependent manner;
   wherein in case the first fusion protein comprises a transcriptional activator, the second fusion protein comprises a DNA-binding protein, and in case the first fusion protein comprises a DNA-binding protein, the second fusion protein comprises a transcriptional activator;
   and further comprising:
(c) a nucleic acid comprising in 5' to 3' direction:
   (i) at least one copy of an operator sequence which can be bound by the DNA-binding protein;
   (ii) a promoter that is responsive to activation by the transcriptional activator; and
   (iii) the coding sequence of the transgene under control of said promoter.

This system of the present invention is characterized in that expression of the transgene can be reversibly activated by illumination with light of a suitable wavelength, and reversibly inactivated by illumination with light of a suitable wavelength.

The term "system" as used herein relates to any entity containing the nucleic acids (a), (b), and (c) as defined above. In particular, said term is intended to encompass any container, plurality of containers, package, kit, vial or the like containing said nucleic acids. Further, the term "system" encompasses any solution, plurality of solutions, complex, plurality of complexes, reaction mixture, biological entity, virus or cell containing said nucleic acids.

The term "transgene" as used herein relates to any gene of interest (goi) expression of which is intended. Said term includes any gene of any origin, provided that said gene can be expressed in the system of the present invention. In one example, the transgene encodes a protein, preferably selected from the group consisting of cytotoxic proteins and unstable proteins, i.e. proteins that are inherently difficult to express in cells. Preferably, the transgene encodes a protein, selected from the group consisting of acid glucosidase, acid-α-glucosidase, angiostatin, aprotinin, bone morphogenetic protein-2 (BMP-2), bone morphogenetic protein-7 (BMP-7), calcitonin, collagen, dornase-α, elastase inhibitor, endostatin, epidermal growth factor (EGF), erythropoietin (EPO), factor VIIa, factor VIII, factor IX, follicle-stimulating hormone, α-galactosidase, glucagon, β-glucocerebrosidase, granulocyte-macrophage colony-stimulating factor, haemagglutinin from H5N1, haemagglutinin from H1N1, hepatitis B antigen (HBsAg), hirudin (thrombin inhibitor), human chorionic gonadotropin (hCG), human glucocerebrosidase, human growth hormone (hGH), human papillomavirus vaccine, human serum albumin, hyaluronidase, iduronate-2-sulfatase, IL-1 receptor antagonist, industrial proteins, insulin, insulin-like growth factor 1 (IGF-1), interferon-α, interferon-β, interleukin 2 (IL-2), interleukin 6 (IL-6), interleukin 10 (IL-10), interleukin 18 (IL-18), kallikrein inhibitor, keratinocyte growth factor, lactoferrin, liposomal acid lipase, lutropin α, lyme disease vaccine, matrix metalloprotease 9 (MMP-9), N-acetylgalactosamine 4 sulfatase, papillomavirus vaccine, parathyroid hormone, platelet derived growth factor (PDGF), pro-insulin, protein C (anti coagulant), recombinant antibodies, recombinant antibody fragments, r-α-L-iduronidase, somatotropin, staphylokinase, thrombin, thyrotropin α, tissue plasminogen activator (t-PA), trypsin, tumor necrosis factor (TNF), tumor necrosis factor α (TNF-α), urate oxidase, and velaglucerase α.

The term "mammalian cell" as used herein encompasses any kind of mammalian cell, provided said mammalian cell is capable of expression of the transgene with the help of the system of the present invention. Preferably, the mammalian cell is selected from the group consisting of CHO cells (Chinese hamster ovary cells), CHO-K1 cells, NIH/3T3 cells, MEF cells (mouse embryonic fibroblast cells), COS-7 cells, HEK293 cells (human embryonic kidney cells), HEK293T cells, PER C6 cells, HUVEC cells, BHK-21 cells, HeLa cells, HT1080 cells, PC12 cells, C2C12 cells, Jurkat cells, K-562 cells, and human embryonic stem cells.

The nucleic acid (a) as defined above can be any nucleic acid comprising the coding sequence of a first fusion protein, said fusion protein comprising a photoreceptor protein, a transcriptional activator or a DNA-binding protein, and a nuclear localization sequence (NLS).

The nucleic acid (b) as defined above can be any nucleic acid comprising the coding sequence of a second fusion protein, said fusion protein comprising a DNA binding protein or a transcriptional activator and a factor that interacts with the photoreceptor protein in a light-dependent manner.

Photoreceptor proteins to be used in the system of the present invention are not particularly limited and include any protein or protein fragment that is capable of undergoing a conformational change in response to absorption of photons of a particular wavelength, and, as a consequence, displays binding to a particular binding partner in a light-dependent manner. In a particular example, the photoreceptor protein is a phytochrome, preferably a phytochrome selected from the group consisting of Phytochrome A (PhyA), Phytochrome B (PhyB), Phytochrome C (PhyC), Phytochrome D (PhyD), and Phytochrome E (PhyE), more preferably PhyB, and most preferably the first 650 amino-terminal amino acids of PhyB. In one particular example, the photoreceptor protein can be PhyB derived from *Arabidopsis thaliana.*

Transcriptional activators to be used in the system of the present invention are not particularly limited and include any protein or protein fragment that displays transcriptional activity in connection with a cognate promoter. In a particular example, the transcriptional activator is selected from the group consisting of acidic transcription activation domains, proline-rich transcription activation domains, serine/threonine-rich transcription activation domains, and glutamine-rich transcription activation domains. Preferably, the transcriptional activator is selected from the group consisting of VP16, e.g. Herpes simplex virus 1-derived VP16, amino acid residues 753 to 881 of GAL4, the p65 domain of NF-κB, amino acid residues 399 to 499 of CTF/NF1, amino acid residues 31 to 76 of AP2, amino acid residues 1 to 427 of ITF1, amino acid residues 2 to 451 of ITF2, amino acid residues 175 to 269 of Oct1, and amino acid residues 132 to 243 of Sp1, wherein VP16 is particularly preferred.

The nuclear localization sequence (NLS) to be used in the system of the present invention is not particularly limited, and suitable NLS are known in the art. As an example, a NLS derived from Simian Virus 40 can be used. Further, suitable NLS can be predicted as described in Nguyen Ba, A. N., A. Pogoutse, et al. (2009), "NLStradamus: a simple Hidden Markov Model for nuclear localization signal prediction." BMC Bioinformatics 10: 202.

DNA-binding proteins to be used in the system of the present invention are not particularly limited and include any protein or protein fragment that is capable of binding to a cognate nucleotide sequence. In a particular example, the DNA-binding protein is selected from the group consisting of members of the TetR family of transcriptional repressors, members of the Lacl family of transcriptional repressors, members of the LexA family of transcriptional repressors, members of the LasR family of transcriptional activators, members of the family of zinc-finger DNA binding domains, and members of the family of Transcription activator-like effector (TALE) DNA binding domains; preferably wherein the DNA-binding protein is TetR.

Factors that interact with the photoreceptor protein in a light-dependent manner to be used in the system of the present invention are not particularly limited and include any proteins or protein fragments that are capable of binding to a cognate photoreceptor protein in a light-dependent manner, *i.e*. that bind to a photo-activated from of the photoreceptor, but not to a photo-inactivated from. In a particular example, said factor is selected from the group consisting of Phytochrome Interacting Factors (PIFs), FHY1/FHL, Phytochrome kinase substrate 1 (PKS1), nucleoside diphosphate kinase 2 (NDPK2), cryptochromes such as CRY1 and CRY2, Aux/IAA proteins, phosphatases such as FyPP and PAPP5, E3 ubiquitin ligases such as COP1, and ARR4. Preferably, said factor is selected from the group consisting of PIF1, PIF2, PIF3, PIF4, PIF5, PIF6, and PIF7, wherein PIF6 is particularly preferred. Even more preferably, said factor consists of the first 100 amino-terminal amino acids of PIF6. In one particular example, said factor can be PIF6 derived from *Arabidopsis thaliana.*

The nucleic acid (c) as defined above comprises in 5' to 3' direction at least one copy of an operator which can be bound by the DNA-binding protein, a promoter that is responsive to activation by the transcriptional activator, and the coding sequence of the transgene to be expressed under control of said promoter.

Operator sequences to be used in the system of the present invention are not particularly limited and include any nucleotide sequence that can be bound by a cognate DNA-binding protein, including synthetic sequences. In a particular example, said operator sequence is selected from the group consisting of the tet operator (tetO), the lac operator, and the lex operator, wherein tetO is particularly preferred. In preferred embodiments of the system of the present invention, nucleic acid (c) as defined above comprises 1 to 100, preferably 5 to 30, more preferably 10 to 16, and most preferably 13 consecutive copies of the operator sequence.

The promoter in nucleic acid (c) which controls expression of the transgene is not particularly limited, provided that it is responsive to activation by the respective transcriptional activator. Suitable promoters are known in the art and include, for example, promoters selected from the group consisting of the minimal human Cytomegalovirus immediate early promoter (p_{hCMVmin}), the minimal Drosophila HSP90 promoter, and the minimal Interleukin-2 promoter.

In a preferred embodiment, nucleic acid (c) as defined above comprises a spacer region downstream of tetO and upstream of the promoter. Said spacer region can have a length of 1 to 1000 basepairs (bp), preferably 250 to 600 bp, more preferably 320 to 390 bp, even more preferably 350 to 360 bp, and most preferably 356 bp. This spacer region is not present in the promoter PhCMV*-1 as originally described in Gossen, M. and H. Bujard (1992), "Tight control of gene expression in mammalian cells by tetracycline-responsive promoters." Proc Natl Acad Sci U S A 89(12): 5547-5551.

In certain embodiments of the system of the present invention, one or two or all of the nucleic acids (a), (b), and (c) as defined above are present in or on one or more vector(s). The term "vector" as used herein relates to any vehicle for the transportation of a nucleic acid into a cell. In particular, said term includes plasmid vectors, viral vectors, cosmid vectors, and artificial chromosomes, wherein plasmid vectors are particularly preferred. Suitable plasmid vectors are known in the art.

In a particularly preferred embodiment, nucleic acids (a) and (b) are present on a first plasmid vector, and nucleic acid (c) is present on a second plasmid vector, the so-called response plasmid. Presence of the nucleic acids (a) and (b) on one plasmid vector results in an advantageous and unexpected increase in transgene expression efficiency. Preferably, in case the nucleic acids (a) and (b) are present on one plasmid vector, the respective coding sequences are separated by an internal ribosome entry site (IRES) and/or a sequence coding for a 2A-peptide. This IRES facilitates translation of both fusion proteins from the same nucleic acid molecule. Suitable IRES sequences and sequences coding a 2A-peptide are known in the art.

Alternatively, in another embodiment of the present invention, one or two or all of the nucleic acids (a), (b), and (c) as defined above are integrated into the genome of the cell. Methods for integrating nucleic acids into the genome of a cell are well known in the art.

In one embodiment of the present invention, the photoreceptor proteins are supplied with their respective chromophore by adding said chromophore to the culture medium. As an example, in the case the photoreceptor protein is a phytochrome, the Phytochromobilin homologue Phycocyanobilin (PCB) from cyanobacteria is added. Alternatively, in another embodiment of the present invention, heme oxigenase (ho1) from cyanobacteria and either Phytochromobilin synthase (mHY2) or PcyA are co-expressed for endogenous production of the chromophore. Accordingly, the system of the present invention may further comprise
(d) a nucleic acid comprising the coding sequences of Heme Oxygenase 1 (ho1); and
(e) a nucleic acid comprising the coding sequence of either Phytochromobilin Synthase (mHY2) or of PcyA.

Preferably, mHY2 is derived from *A. thaliana* and PcyA is derived from the cyanobacterium *Synechocystis.*

In addition to the elements defined above, one, two, three, four or all of the nucleic acids (a), (b), (c), (d) and (e) as defined above may further comprise one or more element(s), selected from the group consisting of linkers, spacers, tags (e.g. an HA-tag), promoters, internal ribosome entry sites (IRES), polyadenylation sequences (pA), such as SV40-derived pA, 2A-peptide, protease cleavage sites, and introns.

In another embodiment of the system of the present invention, said system comprises at least one of the isolated nucleic acid molecule as defined hereinafter, the vector as defined hereinafter, and the host cell as defined hereinafter.

In a further aspect, the present invention relates to an isolated nucleic acid molecule comprising coding sequences of:
(a) a first fusion protein comprising:
   (i) a photoreceptor protein;
   (ii) a transcriptional activator or a DNA-binding protein; and
   (iii) a nuclear localization sequence (NLS);
   and
(b) a second fusion protein comprising:
   (i) a DNA-binding protein or a transcriptional activator; and
   (ii) a factor that interacts with the photoreceptor protein in a light-dependent manner;
wherein in case the first fusion protein comprises a transcriptional activator, the second fusion protein comprises a DNA-binding protein, and in case the first fusion protein comprises a DNA-binding protein, the second fusion protein comprises a transcriptional activator.

In this aspect of the present invention, the first fusion protein, the second fusion protein, the photoreceptor protein, the transcriptional activator, the NLS, the DNA-binding protein, and the factor that interacts with the photoreceptor protein in a light-dependent manner are as defined above for the system of the present invention.

Preferably, the isolated nucleic acid molecule of the present invention further comprises an internal ribosome entry site (IRES) and/or a sequence coding for a 2A-peptide between the coding sequences of the first and the second fusion protein. This IRES facilitates translation of both fusion proteins from the same nucleic acid molecule. Suitable IRES sequences and sequences coding a 2A-peptide are known in the art.

Further, the isolated nucleic acid molecule of the present invention can comprise a suitable promoter upstream of the coding sequences and/or a suitable polyadenylation sequence (pA) downstream of the coding sequences. Suitable promoters and pA sequences are known in the art.

In a further aspect, the present invention relates to a vector comprising the nucleic acid molecule of the present invention. In this aspect, the vector is as defined above for the system of the present invention, wherein plasmid vectors are particularly preferred. Suitable plasmid vectors that can be used in his context are known in the art.

In a further aspect, the present invention relates to a mammalian host cell comprising at least one of the following elements:
(a) the expression system of the present invention;
(b) the nucleic acid molecule of the present invention; and
(c) the vector of the present invention.

In this aspect, the mammalian host cell can be as defined above for the mammalian cell of the system of the present invention.

In a further aspect, the present invention relates to a method for the light-dependent regulation of the transcription of a transgene in a cell, comprising the steps of:
(a) providing a cell comprising a nucleic acid, said nucleic acid comprising in 5' to 3' direction:
   (i) at least one copy of an operator sequence which can be bound by a DNA-binding protein;
   (ii) a promoter that is responsive to activation by a transcriptional activator; and
   (iii) the coding sequence of the transgene under control of said promoter;
(b) expressing in said cell
   a first recombinant fusion protein, comprising:
   (i) a photoreceptor protein;
   (ii) the transcriptional activator or the DNA-binding protein; and
   (iii) a nuclear localization sequence (NLS);
   and a second recombinant fusion protein, comprising:
   (i) the DNA-binding protein or the transcriptional activator; and
   (ii) a factor that interacts with the photoreceptor protein in a light-dependent manner;
   wherein in case the first fusion protein comprises the transcriptional activator, the second fusion protein comprises the DNA-binding protein, and in case the first fusion protein comprises the DNA-binding protein, the second fusion protein comprises the transcriptional activator;
   and
(c) subjecting the cell to light having a suitable wavelength, in order to activate transcription of the transgene; or subjecting the cell to light having a suitable wavelength, in order to deactivate transcription of the transgene.

The present invention further relates to a method for the light-regulated expression of a transgene in a cell, comprising the steps of:
(a) providing a cell comprising a nucleic acid, said nucleic acid comprising in 5' to 3' direction:
   (i) at least one copy of an operator sequence which can be bound by a DNA-binding protein;
   (ii) a promoter that is responsive to activation by a transcriptional activator; and
   (iii) the coding sequence of the transgene under control of said promoter;
(b) expressing in said cell
   a first recombinant fusion protein, comprising:
   (i) a photoreceptor protein;
   (ii) the transcriptional activator or the DNA-binding protein; and
   (iii) a nuclear localization sequence (NLS);
   and a second recombinant fusion protein, comprising:
   (i) the DNA-binding protein or the transcriptional activator; and
   (ii) a factor that interacts with the photoreceptor protein in a light-dependent manner;
   wherein in case the first fusion protein comprises the transcriptional activator, the second fusion protein comprises the DNA-binding protein, and in case the first fusion protein comprises the DNA-binding protein, the second fusion protein comprises the transcriptional activator; and
(c) subjecting said cell to light having a suitable wavelength, in order to activate transcription of the transgene.

In these aspects, the transgene, the mammalian cell, the first recombinant fusion protein, the second recombinant fusion protein, the photoreceptor protein, the transcriptional activator, the NLS, the DNA-binding protein, the factor that interacts with the photoreceptor protein in a light-dependent manner, the nucleic acid, the operator sequence, and the promoter that is responsive to activation by the transcriptional activator, can be as defined above for the system of the present invention.

In a preferred embodiment of the method for the light-regulated expression of a transgene in a mammalian cell of the present invention, said method further comprises the step:
(d) purifying the protein transcribed from the transgene.

Suitable wavelengths of light for the activation or deactivation of transcription of the transgene, i.e. for the activation or deactivation of the photoreceptor protein, are known in the art for each particular photoreceptor protein. As an example, in case the photoreceptor protein is a phytochrome, activation can be effected by light having a wavelength of between 500 and 720 nm, preferably between 620 and 700 nm, and most preferably of about 660 nm. Further, deactivation can be effect by light having a wavelength of more than 720 nm, preferably of about 740 nm. Moreover, it has been shown in Bu, Q., et al., "Dimerization and blue light regulation of PIF1 interacting bHLH proteins in Arabidopsis.", Plant Mol Biol, 2011. 77(4-5): 501-11, that phyrochromes can also be responsive to blue light, e.g. light having a wavelength of about 450 nm.

Means for expressing the recombinant fusion proteins in a cell are not particularly limited and are known in the art. They include for example the transfection of cells with one or more respective expression vectors, e.g. by lipofection, electroporation, viral gene transfer or microinjection. Further, means for transfecting a cell with the nucleic acid carrying the transgene are known in the art. Furthermore, means for purifying a given protein of interest that has been transcribed from the transgene are not particularly limited and are known in the art.

Preferably, the above methods of the present invention are performed in a batch reactor or fed-batch reactor.

As function of photoreceptor proteins is dependent on the presence of a chromophore, the methods of the present invention may further comprise a step of supplementing the chromophore in the cell culture medium, or a step of simultaneously expressing in the cell Heme Oxygenase 1 (ho1), and either Phytochromobilin Synthase (mHY2) or PcyA. Preferably, mHY2 is derived from *A. thaliana* and PcyA is derived from the cyanobacterium *Synechocystis.*

In a further aspect, the present invention relates to a kit, comprising at least one of the following elements:
(a) the isolated nucleic acid of the present invention;
(b) the vector of the present invention; and
(c) the host cell of the present invention.

In preferred embodiments, the kit of the present invention further comprises an isolated nucleic acid comprising in 5' to 3' direction:
(a) at least one copy of an operator sequence which can be bound by a DNA-binding protein;
(b) a promoter that is responsive to activation by a transcriptional activator; and
(c) a cloning site for placing a transgene of interest under control of said promoter.

In this embodiment, the nucleic acid, the operator sequence, and the promoter can be as defined above for the system of the present invention. Further, suitable cloning sites for placing a transgene of interest under control of said promoter are not particularly limited and are known in the art. As an example, cloning sites and techniques for the assembly of DNA molecules are described in Gibson, D. G., L. Young, et al. (2009), "Enzymatic assembly of DNA molecules up to several hundred kilobases." Nat Methods 6(5): 343-345.

The kit of the present invention may further comprise:
(d) a nucleic acid comprising the coding sequences of Heme Oxygenase 1 (ho1); and
(e) a nucleic acid comprising the coding sequence of either Phytochromobilin Synthase (mHY2) or of PcyA.

Alternatively, the kit of the present invention may further comprise:
(f) Phycocyanobilin (PCB).

Moreover, the kit of the present invention may further comprise at least one element, selected from the group consisting of suitable media, suitable cell culture supplements, suitable buffers, suitable transfection reagents, and suitable host cells, which are all not particularly limited and known in the art.

In a further aspect, the present invention relates to a recombinant fusion protein, comprising:
(a) a photoreceptor protein;
(b) a transcriptional activator or a DNA-binding protein; and
(c) a nuclear localization sequence (NLS).

In this aspect, the photoreceptor protein, the transcriptional activator, the DNA-binding protein and the NLS can be as defined above for the system of the present invention.

In a further aspect, the present invention relates to a recombinant fusion protein, comprising
(a) a DNA-binding protein or a transcriptional activator; and
(b) a factor that interacts with a photoreceptor protein in a light-dependent manner.

In this aspect, the DNA-binding protein, the transcriptional activator and the factor that interacts with a photoreceptor protein in a light-dependent manner can be as defined above for the system of the present invention.

In final aspects, the present invention relates to uses of the expression system of the present invention, the isolated nucleic acid molecule of the present invention, the vector of the present invention, the host cell of the present invention, the recombinant fusion proteins of the present invention, or the kit of the present invention for (i) the light-dependent regulation of the transcription of a transgene in a cell, or (ii) the light-regulated expression of a transgene in a cell. The cell may be any mammalian cell as described herein, including non-isolated cells within a human being or an animal. Further, the transgene can be as defined above for the system of the present invention.

Further, the present invention relates to the expression system of the present invention, the isolated nucleic acid molecule of the present invention, the vector of the present invention, the host cell of the present invention, the recombinant fusion proteins of the present invention, or the kit of the present invention for use in medicine, in particular for use in tissue engineering, skin regeneration, tissue regeneration, expression of therapeutic proteins, or induction of blood vessel formation.

Further, the present invention relates to a method for the prophylactic or therapeutic treatment of a condition which can be improved by tissue engineering, skin regeneration, tissue regeneration, expression of therapeutic proteins, or induction of blood vessel formation.

The aim of the present invention was to construct a synthetic, tunable light-regulated gene expression system with rapid ON and OFF kinetics and minimal background. As such, in a particular example of the present invention, the red light induced interaction of Phytochrome B (PhyB) and the Phytochrome-interacting-factor 6 (PIF6) in *A. thaliana* was capitalized on, eliminating the need for a small molecule inducer. PhyB is a chromo-protein that consists of two main domains. The N-terminal domain autoligates its chromophore phytochromobilin (PθB) to form the holo-protein and is the photosensory part of the protein that interacts with PIF6. The C-terminal domain serves regulatory functions. Phytochrome B is synthesized as the biologically inactive P_{R} form. Upon absorption of a red photon, the chromophore isomerizes, triggering a conformational change of the protein into the biologically active P_{FR} form, which can interact with downstream signaling components like PIF6. When the P_{FR}-form absorbs a far-red photon, it reverts back into the inactive P_{R}-form terminating the interaction.

Accordingly, a split transcription factor was constructed by fusing Phytochrome B (PhyB) or an amino-terminal portion thereof to the transcriptional activator VP16 and a nuclear localization sequence. Secondly, the DNA binding domain TetR was fused to the N-terminal part of PIF6 which interacts with the P_{FR} form of PhyB but not with the P_{R} form. The split transcription factor is combined with a response plasmid harboring a TetR-specific operator coupled to a minimal promoter that controls the expression of the target protein. PhyB is supplied with its chromophore by adding the Phytochromobilin homologue Phycocyanobilin (PCB) from cyanobacteria to the culture medium. Alternatively, heme oxigenase (ho1) from cyanobacteria and Phytochromobilin synthase (mHY2) from *Arabidopsis thaliana* are co-expressed for endogenous production of the chromophore. Illumination with red light triggers the heterodimerization of TetR-PIF6 with PhyB-VP16-NLS at the promoter site of the response plasmid, resulting in expression of the target protein. Illumination with far red light reverses this interaction and shuts off transgene expression. For the production of proteins, the PhyB and PIF6-derived split transcription factor as well as the response plasmid and optionally the enzymes for Phytochromobilin synthesis can be integrated into the genome of a production cell line. In a batch/fed-batch production process, this makes it possible to grow the cells to an optimum density while transgene expression is suppressed either by keeping the reactor in the dark, or by illumination with far red light, or by omitting the chromophore. In the second phase, transgene expression is induced by illumination with red light, resulting in massive production of the target protein within a short time frame prior to harvesting and purification. In this way it is possible to minimize adverse effects arising from the prolonged presence of the target protein in the production system. These adverse effects can range from cytotoxicity to unfolding or degradation of the manufactured protein.

In a proof-of-principle demonstration, the applicability of the system of the present invention *in vivo* by a cell-therapy like approach for light inducible vascularization in chicken embryos could be shown. It was concluded that the light-regulated gene expression described herein can be applied in any mammalian cell or organism accessible to light, ranging from single cells to complex organisms, with the superior tissue penetrating characteristics of red light being of particular importance for the latter application.

The figures show:

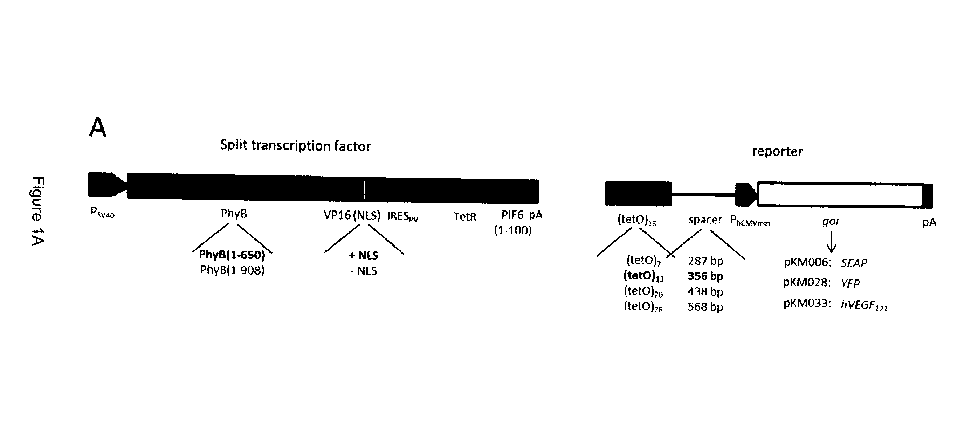

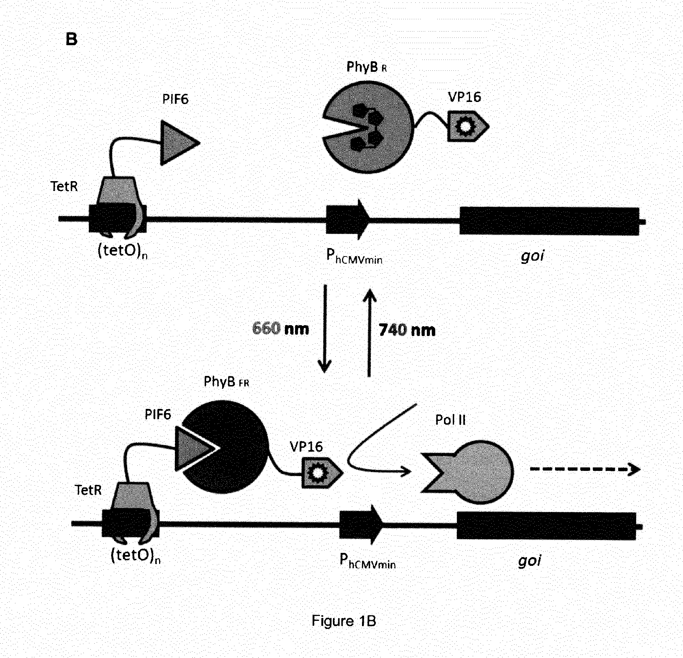

Figure 1: Schematic representation of the red light inducible transgene expression system.
   **(A)** Configuration of the system. The split transcription factor comprising the PIF6 and PhyB fusion proteins is expressed from a bicistronic expression unit and directs red light induced transgene expression from the reporter plasmid. The sites of optimization are indicated and the optimized configuration is marked in bold letters. The optimized split transcription factor is encoded on pKM022.
   **(B)** Mode of function. In far-red light (740 nm) PhyB_{R} does not interact with PIF6 (1-100) that is tethered to the tetO-motif of the response construct via the DNA binding domain TetR. Upon absorption of a red photon (660 nm) PhyB_{FR} interacts with PIF6 bringing the transactivator domain VP16 into close proximity of the P_{hCMVmin} minimal promoter, thus enabling transcription of the gene of interest (goi) by RNA polymerase II. This process is completely reversed by the absorption of a far-red photon.

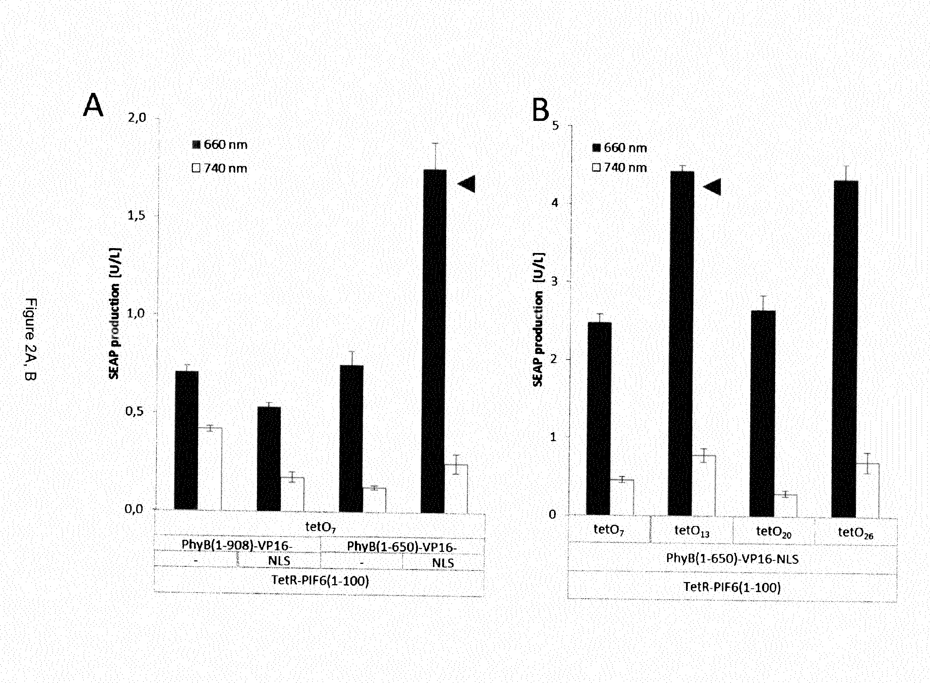

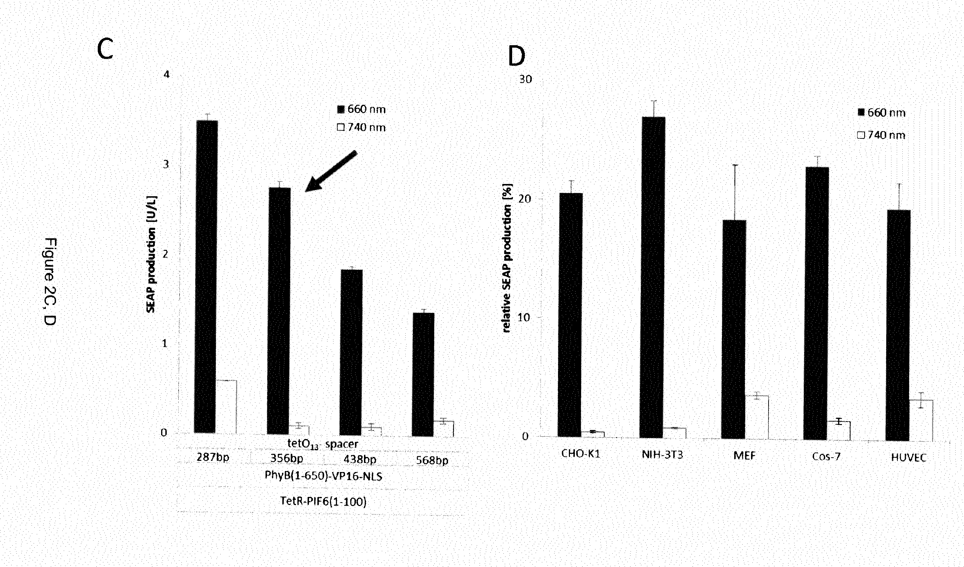

Figure 2: Optimization of the light inducible transgene expression system in CHO-K1 cells and validation in mammalian cells.
   (A) Effect of PhyB variant (908 or 650 amino acids) and the absence or presence of an NLS on PhyB-VP16.
   (B) Test of various tetO multimers in the reporter construct.
   **(C)** Assessment of various spacer-lengths between the tet-operator and the minimal promoter.
   **(D)** Validation of the system in various mammalian cells. Arrowheads mark the configuration that was further optimized and the arrow indicates the combination with the best expression performance that was used in all further experiments. Data are means of three independent experiments and error bars indicate the standard deviations. The data in **(D)** are normalized to the values obtained from cells transfected in parallel with the reporter and a vector for constitutive expression of TetR-VP16 (pSAM200), in order to correct for transfection efficiency.

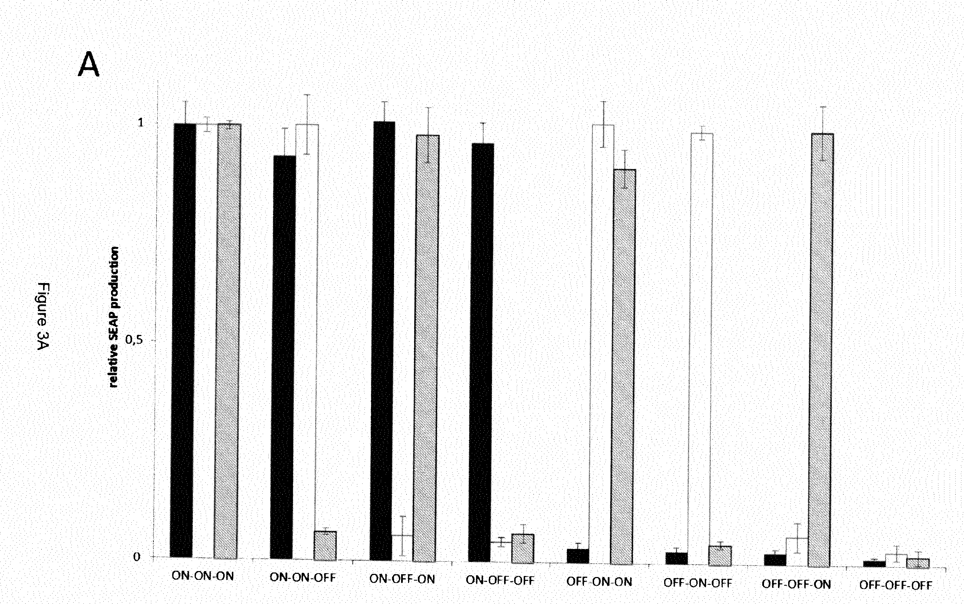

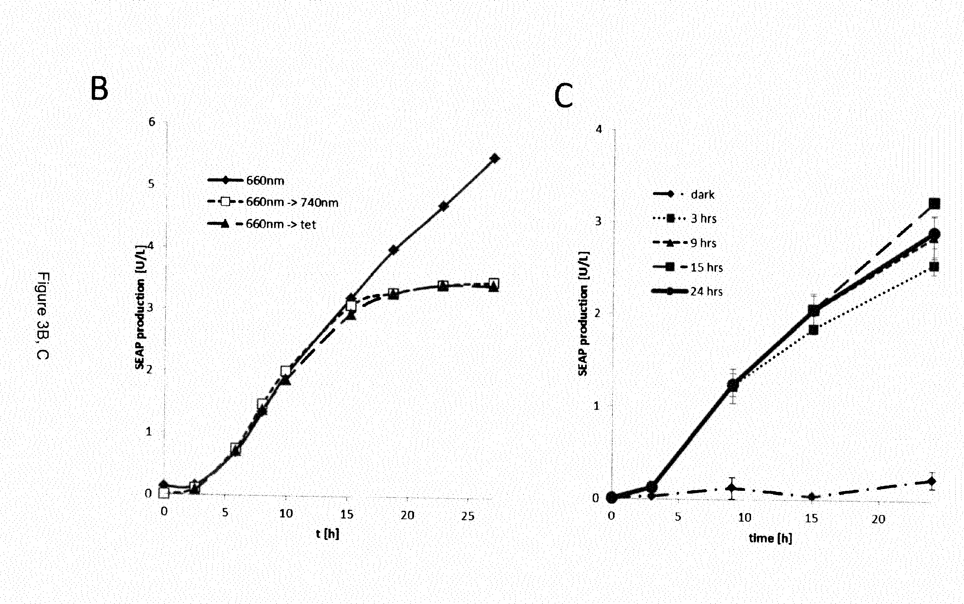

Figure 3: Reversibility of the light inducible transgene expression system.
   **(A)** CHO-K1 cells were transfected with a SEAP reporter (pKM006) and the split transcription factor (pKM022) and illuminated with red (ON) or far-red (OFF) light for periods of 24 h. After each period the culture medium was replaced with fresh medium and the cells were illuminated as indicated. SEAP activity in the supernatants was measured and the data were normalized to the ON-ON-ON configuration.
   **(B)** OFF-kinetics. CHO-K1 cells were illuminated for 6 h with red light. Then the cells were either illuminated with red light, far-red light or tetracycline was added. Samples were taken at the indicated time points and SEAP activity was measured.
   **(C)** SEAP expression after illumination shut-off. CHO-K1 cells were grown in red light and put in the dark after 3, 9, 15 or 24 h. Control cells were left in the dark for the whole experiment. At the indicated time points samples were collected and SEAP activity was measured. Data are means of four independent experiments and error bars indicate the standard deviations **(A, C). (B)** shows data from one experiment.

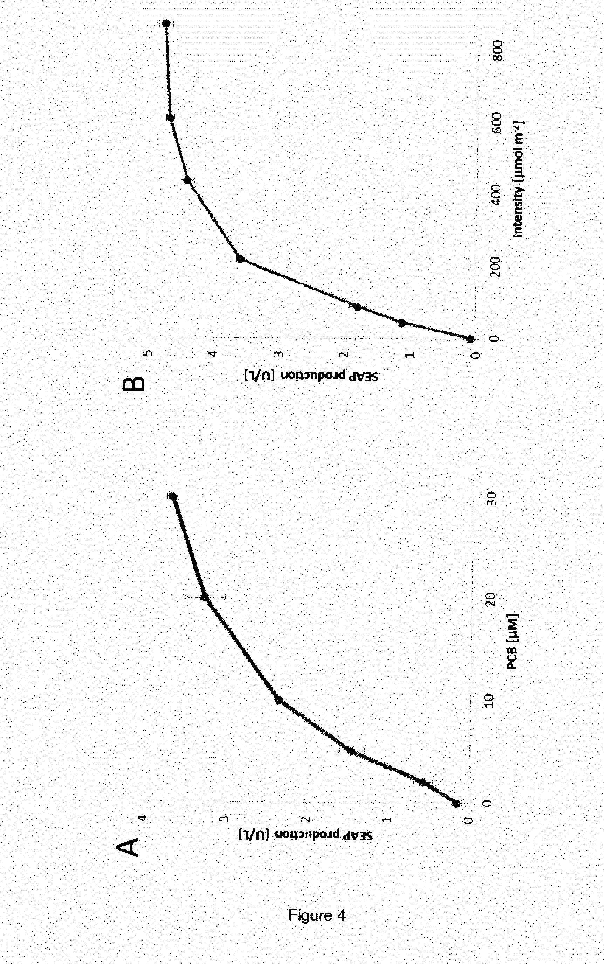

Figure 4: Adjustable reporter gene expression.
   **(A)** Adjustable SEAP production by varying PCB concentrations. After transfection of CHO-K1 with a SEAP reporter (pKM006) and the split transcription factor (pKM022), varying amounts of PCB were added to the cell prior to illumination with 660 nm light for 24 h.
   **(B)** Fluence-response curve of reporter expression. 25 min pulses of varying intensity were applied, followed by incubation in the dark for 24 h. After 24 h of illumination, SEAP activity in the supernatants was measured. Data are means of four independent experiments and error bars indicate the standard deviations.
Figure 5: Space resolved gene expression.
   CHO-K1 cells were transfected with a mcherry reporter (pKM078) and the split transcription factor (pKM022) and illuminated with red light through photomasks (left image) for 1 h (0.5 µmol m⁻²s⁻¹). Pictures were taken after 23 hours incubation in the dark (right image). Scale bar, 1 cm.
Figure 6: Red light controlled hVEGF₁₂₁ production.
   One million CHO-K1 cells transiently transfected with the light inducible split transcription factor (pKM022) and a hVEGF₁₂₁ reporter (pKM033) were illuminated with 660 nm or 740 nm light for 24 h and hVEGF₁₂₁ production was quantified.
Figure 7: Red light controlled vascularization in chicken embryos.
   One million CHO-K1 cells transiently transfected with the light inducible split transcription factor (pKM022) and a hVEGF₁₂₁ reporter (pKM033), were applied onto the CAMs of 10 day old chicken embryos and illuminated with **(A)** 660 nm or **(B)** 740 nm light for 2 days (until embryonic day 12). The pictures are representative still video images of the growing CAM microvasculature at embryonic day 12 following intravenous injection of FITC-dextran (top panels overview; lower panels insets at higher magnification). Markers of an angiogenic response as exemplified by (i) the increased number of the feeding vessels; (ii) and atypical (brush- and delta-like) endpoint patterns (arrowheads) and (iii) their tortuous shape (arrows) are indicated.

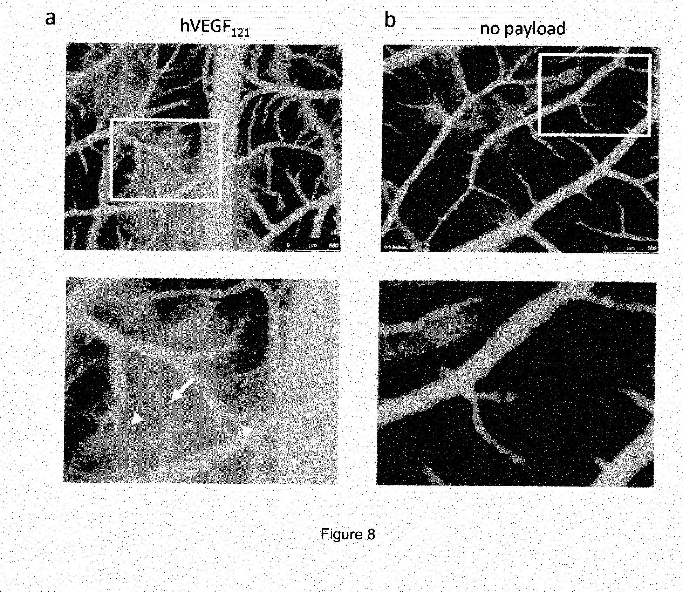

Figure 8: hVEGF₁₂₁ induced vascularization of chicken embryos.
   **(A)** Effect of 1 µg hVEGF₁₂₁ in a PEG-hydrogel on the CAM vasculature. hVEGF₁₂₁ was incorporated in a PEG-hydrogel and applied on the CAM of 10 day old chicken embryos.
   **(B)** Control with empty PEG-gels. The pictures are representative still video images of the growing CAM microvasculature at embryonic day 12 following intravenous injection of FITC-dextran (top panels overview; lower panels insets at higher magnification). Markers of an angiogenic response as exemplified by (i) the increased number of the feeding vessels; (ii) a brighter capillary plexus indicating a higher density, (iii) atypical (brush- and delta-like) endpoint patterns (arrowheads) and (iii) their tortuous shape (arrows) are indicated.
Figure 9: Comparison of the ligiht-inducible transgene expression system of the present invention to US-Patent 6,887,688 B2.
   **(A)** Plasmids used. In accordance with the above patent PhyA from *Oryza sativa* or PhyB from *Arabidopsis thaliana* was fused to the DNA-binding domain TetR and the transactivation domain VP16 on pKM070 or pMZ320, respectively. The light inducible split-transcription factor of the present invention is produced from pKM022, pMZ324 codes for PIF6(1-100) with a nuclear localization sequence (NLS) and pKM006 is the SEAP reporter.
   **(B)** Performance of the light-inducible gene expression systems. For the light-inducible system pKM022 was co-transfected with pKM006. For comparison with the US patent pKM070 or pMZ320 was co-transfected with pKM006 and in additional experiments pMZ324 was added to these configurations. The experiments were conducted in CHO-K1 cells. 24 hours post transfection the culture medium was replaced and the cells were exposed to red or far-red light. Reporter gene assay was performed another 24 hours later.

The present invention will now be further illustrated in the following examples without being limited thereto.

### Examples

### Experimental procedures:

*DNA cloning.* pKM006 (tetO₁₃-356bp-SEAP-pA), pKM028 (tetO₁₃-356bp-YFP-pA) and pKM033 (tetO₁₃-356bp-hVEGF₁₂₁-pA) are response plasmids that allow red light induced reporter expression in conjunction with pKM022 (P_{SV40}-PhyB(1-650)-VP16-NLS-IRES_{PV}-TetR-PIF6(1-100)-HA-pA) that encodes constitutive expression of the red light-dependent transcription factor. Detailed information on expression vector design and plasmids used in this study, as well as chemically synthesized nucleotide sequences used therein, is provided in Tables 1 and 2, respectively.

**Table 1: Expression vectors and oligonucleotides designed and used in this study**

| **Plasmid** | **Description** |
|---|---|
| pKM001 | Vector encoding SEAP under the control of P_{Tet} harboring a 287 bp spacer between the heptameric tetO operator and the minimal promoter (tetO₇-287bp-P_{hcMVmin}-SEAP-pA) |
| | tetO₇-287bp-P_{hcMVmin} was chemically synthesized (Supplementary table 2) and liqated (*Aat*II/EcoRI) into pMK82. |
| pKM002 | Vector encoding SEAP under the control of a modified P_{Tet} harboring a 287 bp spacer between the 13-mer tetO operator and the minimal promoter (tetO₁₃-287bp-P_{hcMVmin}-SEAP-pA) |
| | tetO₁₃-287bp-P_{hCMVmin} was chemically synthesized (Supplementary table 2) and liqated (*Aat*II/EcoRI) into pMK82. |
| pKM003 | Vector encoding SEAP under the control of a modified P_{Tet} harboring a 287 bp spacer between the 20-mer tetO operator and the minimal promoter (tetO₂₀-287bp-P_{hcMVmin}-SEAP-pA) |
| | tetO₁₃-287bp-P_{hCMVmin} was excised (*Eco*RV/*Eco*RI) from pKM002 and liqated (*Nru*I/*Eco*RI) into pKM001. |
| pKM004 | Vector encoding SEAP under the control of a modified P_{Tet} (tetO₂₆-287bp-P_{hCMVmin}-SEAP-pA) |
| | tetO₂₀-287bp-P_{hCMvmin} was excised (EcoRV/EcoRI) from pKM002 and ligated (*Nru*I/*Eco*RI) into pKM002. |
| pKM006 | Vector encoding SEAP under the control of a modified P_{Tet} harboring a 356 bp spacer between the 13-mer tetO operator and the minimal promoter (tetO₁₃-356bp-P_{hCMVmin}-SEAP-pA) |
| | A 372 bp fragment was amplified from CFP using oligos oKM001: 5'-caagtacctqcaggCCCTGAAGTTCATCTGCACC-3' and oKM003: caagtcgctagcTCTTGAAGTTGGCCTTGATGC, digested (*Sbf*I/*Nh*eI) and ligated (*Sbf*I/*Nhe*I) into pKM002. |
| pKM010 | Vector encoding SEAP under the control of a modified P_{Tet} harboring a 438 bp spacer between the 13-mer tetO operator and the minimal promoter (tetO₁₃-438bp-P_{hCMVmin}-SEAP-pA) |
| | A 438 bp fragment was amplified from CFP using oligos oKM002: 5'-caagtacctacaggACGTAAACGGCCACAAGTTC-3' and oKM003: 5'-caagtcgctagcTCTTGAAGTTGGCCTTGATGC-3', digested (*SbfI*/*Nhe*I) and liqated (*Sbf*I/*Nhe*I) into pKM002. |
| pKM014 | Vector encoding SEAP under the control of a modified P_{Tet} harboring a 568 bp spacer between the 13-mer tetO operator and the minimal promoter (tetO₁₃-568bp-P_{hCMVmin}-SEAP-pA) |
| | A 568 bp fragment was amplified from CFP using oligos oKM002: 5'-caagtacctgcaggACGTAAACGGCCACAAGTTC-3' and oKM004: 5'-caagtcgctagcTCTTTGCTCAGCTTGGACTG-3', digested (*Sbf*I/*Nhe*I) and ligated (*Sbf*I/*Nhe*I) into pKM002. |
| pKM017 | Vector encoding a P_{SV40}-driven PhyB(1-908)-VP16-NLS expression unit (P_{SV40}-PhyB(1 -908)-VP16-NLS-pA) |
| | VP16-NLS was amplified from pMK216 using oligos oKM007: 5'-atcagtgaattcGATAGTGCTGGTAGT-3 and oKM006: 5'-atcagttctactatcacaccttccgctttttcttgggCCCACCGTACTCGTCAATTC-3', diqested (*Eco*RI/*Xba*I) and ligated (*Eco*RI/*Xba*I) into pMK216. |
| pKM018 | Vector encoding a P_{SV40}-driven PhyB(1-650)-VP16-NLS expression unit (P_{SV40}-PhyB(1-650)-VP16-NLS-pA) |
| | VP16-NLS was excised (*Eco*RI/XbaI) from pKM017 and ligated (*Eco*RI/*Xba*I) into pMK233. |
| pKM019 | Bicistronic vector encoding PhyB(1-908)-VP16 and TetR-PIF6(1-100)-HA under control of P_{SV40} (P_{SV40}-PhyB(1-908)-VP16-IRES_{PV}-TetR-PIF6(1-100)-HA-pA) |
| | IRES_{PV} was excised (*Pst*I/*Not*I) from pMK082, PhyB(1-908)-VP16 was excised (*Aat*II/*Pst*I) from pMK216 and both fragment were ligated (*Aat*II/*Not*I) into pMK235. |
| pKM020 | Bicistronic vector encoding PhyB(1-908)-VP16-NLS and TetR-PIF6(1-100)-HA under control of P_{SV40} (P_{SV40}-PhyB(1-908)-VP16-NLS-IRES_{PV}-TetR-PIF6(1-100)-HA-pA) |
| | P_{SV40}-PhyB(1-908)-VP16-NLS was excised (*Aa*tII/*Pst*I) from pKM017 and ligated (*AatII*/*Pst*I) into pKM019. |
| pKM021 | Bicistronic vector encoding PhyB(1-650)-VP16 and TetR-PIF6(1-100)-HA under control of P_{SV40} (P_{SV40}-PhyB(1-650)-VP16-IRES_{PV}-TetR-PIF6(1-100)-HA-pA) |
| | IRES_{PV} was excised (*Pst*I/*Not*I) from pMK082, PhyB(1-650)-VP16 was excised (*Aat*II/*Pst*I) from pMK233 and both fragment were ligated (*AatII*/*Not*I) into pMK235. |
| pKM022 | Bicistronic vector encoding PhyB(1-650)-VP16-NLS and TetR-PIF6(1-100)-HA under control of P_{SV40} (P_{SV40}-PhyB(1-650)-VP16-NLS-IRES_{PV}-TetR-PIF6(1-100)-HA-pA) |
| | P_{SV40}-PhyB(1-650)-VP16-NLS was excised (*Aa*tII/*Pst*I) from pKM018 and ligated (A*at*II/*Pst*I) into pKM021. |
| pKM028 | Vector encoding the destabilized EYFP variant d2EYFP under the control of a modified P_{Tet} (tetO₁₃-356bp-P_{hCMVmin}-d2EYFP-pA) tetO₁₃-356bp-P_{hCMVmin} was excised (SspI/EcoRI) from pKM006 and ligated (SspI/EcoRI) into pLMK164. |
| pKM033 | Vector encoding hVEGF₁₂₁ under the control of a modified P_{Tet} (tetO₁₃-356bp-P_{hCMVmin}-hVEGF₁₂₁-pA) |
| | hVEGF₁₂₁ was amplified using oligos oKM015: 5'-caagtcgaattcaccgCCATGAACTTTCTGCTGTCTTG-3' and oKM016: 5'-ctgaacgcgaccacTCACCGCCTCGGCTTGTC-3', digested (*Eco*RI/*Not*I) and liqated (*Eco*RI/*Not*I) into pKM028. |
| pKM078 | Vector encoding mcherry under control of a modified P_{Tet} (tetO₁₃-356bp-P_{hCMVmin}-mcherry-pA) |
| | MCherry was excised (*Bam*HI/*Not*I) from pMK047 and ligated (*Bam*HI/*No*t) into pKM028). |
| pLMK164 | Vector encoding the destabilized EYFP variant d2EYFP under the control of P_{Tet}(P_{Tet}-d2EYFP-pA) |
| | dEYFP was excised (*Eco*RI/*Not*I) from pd2EYFP (Clontech) and ligated (*Eco*RI/*Not*I) into pMF111. |
| pMF111 | Vector encoding a P_{Tet}-driven SEAP expression unit (P_{Tet}-SEAP-pA) |
| pMK047 | Vector encoding a P_{EF1a}-driven mcherry expression unit (P_{EF1a} mCherry-pA) |
| | mcherry was amplified using oligos oMK057: 5'-ccaccactagtccaccATGGTGAGCAAGGGCGAGGAGG-3' and oMK58: 5'-ggtgtgcggccgctcatatgtcctgtgagggttgaattcCTTGTACAGCTCGTCCATG CCGCCG-3', digested (*Spe*I/*Not*I) and ligated (*Spe*I/*Not*I) into pWW029. |
| pMK082 | Vector encoding SEAP under the control of P_{Tet} (tetO₇-287bp-P_{hCMVmin}-SEAP-pA) |
| | SEAP was excised (*Eco*RI/*Not*I) from pMF111 and ligated (*Eco*RI/*Not*I) into pWW927. |
| pMK216 | Vector encoding a P_{SV40}-driven PhyB(1-908)-VP16 expression unit (P_{SV40}-PhyB( 1-908)-VP16-pA) |
| | PhyB (1-908) was amplified from pAL149²¹ using oligos oMK246: 5'-caccgcagccgcCCACCATGGTTTCCGGAGTCGGGGGTAG-3' and oMK247: 5'-ggtggcacgcGGCTGTACGCGGAACCAGCACTACCAGCACTACCAG-3', digested (*Not*I/*Bss*HII) and ligated (*Not*I/*Bss*HII) into pSAM200. |
| pMK233 | Vector encoding a P_{SV40}-driven PhyB(1-650)-VP16 expression unit (P_{SV40}-PhyB(1-650)-VP16-pA) |
| | PhyB (1-650) was amplified from pAL149²¹ using oligos oMK246: 5'-caccgcaaccgcCCACCATGGTTTCCGGAGTCGGGGGTAG-3' and oMK280: 5'-tacagaattcACCTAACTCATCAATCCCCTGTTCCC-3', diqested (*Not*I/*Eco*RI) and liqated (*Not*I/*Eco*RI) into pMK216. |
| pMK235 | Vector encoding a P_{SV40}-driven TetR-PIF6(1-100)-HA expression unit (PSV40-TetR-PIF6(1-100)-HA-pA) |
| | PIF6(1-100)-HA was amplified from pAL175²¹ using oligos oMK281: 5'-ccacacgcgctcgatagtgctggtagtgctggtagtgctggtATGATGTTCTTACCAAC CGATTATTGTTGC-3' and oMK282: 5'-tactaagcttttaagcgtaatctggaacatcgtatgggtaGTCAACATGTTTATTGCTTT CCAACATGTTTG-3', digested (*Bss*HII/*Hin*dIII) and ligated (*Bss*HII/*Hin*dIII) into pSAM200. |
| pRSet | P_{T7}-driven bacterial expression vector |
| pSAM200 | Constitutive TetR-VP16 expression vector (P_{SV40}-TetR-VP16-pA) |
| pWW029 | Vector encoding the erythromycin repressor protein E under control of P_{EFIa} (P_{EFIa}-E-pA) |
| pWW927 | Vector encoding SEAP under the control of P_{Tet} (tetO₇-ETR₈-P_{hCMVmin}-biotinidase-pA) |

| | |
|---|---|
| CFP, cyan fluorescent protein; E, erythromycin repressor protein; ETR₈, operator sequence binding E; EYFP, enhanced yellow fluorescent protein; d2EYFP, destabilized enhanced yellow fluorescent protein with a half-life of 2 h ; HA, human influenza hemagglutinin derived epitope tag; P_{hCMVmin}, minimal human cytomegalovirus immediate early promoter; hVEGF₁₂₁, 121 amino acids splice variant of human vascular endothelial growth factor; IRES_{PV}, polioviral internal ribosome entry site; NLS, nuclear localization signal from simian virus 40 large T antigen; pA, polyadenylation signal; PhyB, Phytochrome B; PhyB(1-650), N-terminus of Phytochrome B with amino acids 1-650; PhyB(1-908), N-terminus of Phytochrome B with amino acids 1-908; P_{EF1a}, human elongation factor 1α promoter; PIF6, Phytochrome-interacting-factor 6; PIF6(1-100), N-terminus of Phytochrome-interacting-factor 6 with amino acids 1-100; P_{SV40}, simian virus 40 early promoter; P_{Tet}, tetracycline-responsive promoter; SEAP, human placental secreted alkaline phosphatase; tetO, operator sequence binding TetR; TetR, tetracycline repressor protein; VP16, *Herpes simpex* virus-derived transactivation domain; YFP, yellow fluorescent protein Uppercase in oligos, annealing sequence; underlined sequence, restriction site. | |

**Table 2: Nucleotide sequences of chemically synthesized DNA**

| **Designatio n** | **Nucleotide Sequence** |
|---|---|
| tetO₇-287bp-P_{hCMVmin} | 5'- ...gacgtcgtcgacgatatccctcgaTCGAGTTTACCACTCCCTATCAGTGATAGAG AAAAGTGAAAGTCGAGTTTACCACTCCCTATCAGTGATAGAGAAAAGTGAAAGTCGAG TTTACCACTCCCTATCAGTGATAGAGAAAAGTGAAAGTCGAGTTTACCACTCCCTATC AGTGATAGAGAAAAGTGAAAGTCGAGTTTACCACTCCCTATCAGTGATAGAGAAAAGT GAAAGTCGAGTTTACCACTCCCTATCAGTGATAGAGAAAAGTGAAAGTCGAGTTTACC ACTCCCTATCAGTGATAGAGAAAAGTGAAAGtcgagctcggtacgctatggcatgcat gtgtcgcgacctgcaggtaggcgtgtacggtgggaggcctaggcttttgcaaaaagct tcgaagtttaaacgattgaatataaccgacgtgactgttacatttagggattgaatat aaccgacgtgactgttacatttagggattgaatataaccgacgtgactgttacattta gggattgaatataaccgacgtgactgttacatttagggataaacgattgaatataacc gacgtgactgttacatttagggattgaatataaccgacgtgactgttacatttaggga ttgaatataaccgacgtgactgttacatttagggattgaatataaccgacgtgactgt tacatttagggatgctagcCCTATATAAGCAGAGCTCGTTTAGTGAACCGTCAGATCG CCTGGAGACGCCATCCACGCTGTTTTGACCTCCATAGAAGACACCGGGACCGATCCAG CCTccgcggccccggtaccgaattc...-3' |
| tetO₁₃-287bp-P_{hCMVmin} | 5′- ...gacgtcgtcgacgatatcCCTCGATCGAGTTTACCACTCCCTATCAGTGATAGAG AAAAGTGAAAGTCGAGTTTACCACTCCCTATCAGTGATAGAGAAAAGTGAAAGTCGAG TTTACCACTCCCTATCAGTGATAGAGAAAAGTGAAAGTCGAGTTTACCACTCCCTATC AGTGATAGAGAAAAGTGAAAGTCGAGTTTACCACTCCCTATCAGTGATAGAGAAAAGT GAAAGTCGAGTTTACCACTCCCTATCAGTGATAGAGAAAAGTGAAAGTCGAGTTTACC ACTCCCTATCAGTGATAGAGAAAAGTGAAAGTCGAGTTTACCACTCCCTATCAGTGAT AGAGAAAAGTGAAAGTCGAGTTTACCACTCCCTATCAGTGATAGAGAAAAGTGAAAGT CGAGTTTACCACTCCCTATCAGTGATAGAGAAAAGTGAAAGTCGAGTTTACCACTCCC TATCAGTGATAGAGAAAAGTGAAAGTCGAGTTTACCACTCCCTATCAGTGATAGAGAA AAGTGAAAGTCGAGTTTACCACTCCCTATCAGTGATAGAGAAAAGTGAAAGTCGAGCT CGGTACGCTATGGCATGCATGTGtcgcgacctgcaggtaggcgtgtacggtgggaggc ctaggcttttgcaaaaagcttcgaagtttaaacgattgaatataaccgacgtgactgt tacatttagggattgaatataaccgacgtgactgttacatttagggattgaatataac cgacgtgactgttacatttagggattgaatataaccgacgtgactgttacatttaggg ataaacgattgaatataaccgacgtgactgttacatttagggattgaatataaccgac gtgactgttacatttagggattgaatataaccgacgtgactgttacatttagggattg aatataaccgacgtgactgttacatttagggatgctagcCCTATATAAGCAGAGCTCG TTTAGTGAACCGTCAGATCGCCTGGAGACGCCATCCACGCTGTTTTGACCTCCATAGA AGACACCGGGACCGATCCAGCCTccgcggccccggtaccgaattc...-3′ |
| Upper case in 5' -> 3' direction, n-mer tetO-operator and P_{CMVmin} minimal promoter, underlined sequences in 5' -> 3' direction, restriction sites *Aat*II, *Eco*RV. *Nru*I, *Eco*RI | |

Purification of phycocyanobilin (PCB) from *Spirulina.* PCB was purified from *Spirulina* by a modification of a protocol known in the art. In particular, 50 g of *Spirulina* powder were boiled with 500 ml methanol for 1-2 min. After cooling, the liquid was aspirated and the procedure was repeated 6-8 times, until the flow-through became colorless. From this point onwards, all glassware was wrapped in aluminum-foil and all steps were carried out under low-light conditions using a green safe light, in order to protect the free chromophore.

For methanolysis, the powder was boiled in 500 ml of methanol for 4 h, the liquid was aspirated and reduced in a rotary evaporator to 30 ml, mixed with 70 ml of diethyl ether and extracted with 100 ml of 1 % (w/v) citric acid. The aqueous phase was collected for a second extraction, while PCB was extracted from the organic phase with 50 ml 1 % (w/v) sodium hydrogen carbonate. The organic phase was discarded and the aqueous phase was extracted with 50 ml chloroform, after addition of 10 ml 1 M HCl for protonation of PCB. The aqueous phase from the first extraction step was extracted in the same way and the two PCB-containing chloroform fractions were combined. After all chloroform had been evaporated in a rotary evaporator, PCB was dried overnight, dissolved in 1.5 ml DMSO and stored at -20 °C. The PCB concentration was determined photometrically as known in the art and was typically 15-25 mM.

*Cell culture and light experiments*. CHO-K1 were cultured in HTS-medium (Cell Culture Technologies) supplemented with 10 % FBS, 2 mM L-glutamine (Sigma). COS-7, HEK-293T and MEF were maintained in DMEM supplemented with 10 % FBS. NIH-3T3 cells were cultured in DMEM with 10 % newborn calf serum (PAN). 100 U/ml penicillin and 0.1 mg/ml streptomycin (PAN) was added to all culture media and all cells were cultured at 37 °C, 5 % CO₂. CHO-K1, Cos-7, MEF and NIH-3T3 cells were transfected using a polyethylenimine (PEI, linear, MW: 25.000 Da) (Polyscience) based method. In brief, a 1 M PEI solution in water was adjusted to pH 7.0 with HCl, then sterile filtered and stored at -80°C in aliquots. 30,000 - 70,000 cells were seeded per well of a 24-well plate and cultivated overnight. Per well 0.75 µg of DNA were diluted in 50 µl OptiMEM (Invitrogen) and mixed with 2.5 µl PEI in 50 µl OptiMEM by vortexing. After 15 min incubation at room temperature the precipitate was added to the cells. For other plate formats, the cell number and amount of reagents was scaled up according to the growth area. For a 100 mm dish, 15 µg DNA and 50 µl PEI were diluted in 500 µl OptiMEM each. For CHO-K1 cells the culture medium was replaced 5 h after the transfection in order to minimize cytotoxic effects, while the DNA remained on the other cell types overnight. HEK-293T cells were transfected using a calcium phosphate based method. In brief, 70,000 cells were seeded into each well of a 24-well plate and cultured overnight. 0.75 µg of plasmid DNA in 20 µl H₂O containing 250 mM CaCl₂, was mixed with 20 µl 2×HBS solution (50 mM HEPES, 280 mM NaCl, 1.5 mM Na₂HPO₄, pH 7.1). Following incubation for 15 min at room temperature, the precipitate was added to the culture medium and the plates were centrifuged (5 min at 1200 x g). The culture medium was replaced 5 h post-transfection. For light experiments the red light dependent transcription factor plasmid and the reporter plasmid were transfected in a ratio of 2:1 and the culture medium was replaced with medium containing 15 µM PCB 24 h post-transfection. All experimental procedures after the addition of PCB were carried out under green LED light. Before illumination with 660 nm (8 µmol m⁻² s⁻¹ unless indicated) or with 740 nm (3.5 µmol m⁻² s⁻¹) light from LED arrays, the cells were incubated in the dark for 1 h. Light-intensity was adjusted using neutral density filters (Schott) that were placed on top of the culture dishes. When indicated, tetracycline was added to the cells at a concentration of 3 µg ml⁻¹ from a 3 mg ml⁻¹ stock solution in ethanol. Where not indicated elsewise, for temporal control of gene expression, cells were transfected with the red light dependent transcription factor (pKM022) in combination with a SEAP reporter (pKM006) or with a VEGF reporter (pKM033) and illuminated from above. To spatially control gene expression, single cell layers were transfected with the red light dependent transcription factor (pKM022) and a mcherry-reporter (pKM078). A photomask was stuck to the bottom of the culture plate and the plate was illuminated from the bottom with 660 nm light (0.5 µmol m⁻² s⁻¹) for 1 h followed by incubation in the dark for 24 h.

*SEAP and VEGF assays.* SEAP in cell culture supernatants was quantified as known in the art. VEGF was quantified using a human VEGF-ELISA kit (Peprotech).

*Chorio-Allantoic-Membrane (CAM) assay.* 1.2 million CHO-K1 cells were seeded in a 100 mm dish and transfected after 24 h with the red light dependent transcription factor (pKM022) and a hVEGF₁₂₁-reporter (pKM038). After 24 h PCB was added to a final concentration of 15 µM and the cells were incubated in the dark for 1 h. The cells were trypsinized, and 1 million cells were incorporated into a polyethylene glycol (PEG) gel. The 3% PEG hydrogels were formed as known in the art, by Factor XIII (FXllla)-catalyzed crosslinking of 8-arm Lys-MMP-PEG and TG-MMP-PEG in Tris buffer (50 mM Tris, pH 7.6) containing 50 mM CaCl₂ and 50 µM Lys-RGD. One million transfected CHO-K1 cells were added for a total gel volume of 20 µL and the cross-linking reaction was performed for 30 min at 37 °C and 5 % CO₂. To obtain the chicken CAM, broiler eggs (Ernst Wüthrich Brüterei) were first incubated for 3 days at 37°C. Then they were carefully opened and poured into 100mm petri dishes for *ex-ovo* culture of the chicken embryos. The developing chicken embryos were incubated at 37°C in a humid atmosphere. After 9 days of incubation, the PEG hydrogels containing 1 million of the transfected CHO-K1 cells were deposited on the surface of the CAM. These were placed back in the incubator for 2 more days and illuminated as indicated. On day 11 after incubation, *in vivo* microscopy was performed by injecting 100 µL of 2.5% FITC-Dextran (Sigma) in the CAM vasculature. The vasculature was assessed for morphological changes in the vessel structure and capillary plexus.

### Example 1:

### Design and fine tuning of the red light inducible gene expression system.

The red light inducible expression system of the present invention was designed based on the concept of split transcription factors, where two proteins are fused to a DNA-binding domain and a transactivation domain respectively. Only upon interaction of these fused proteins, the transcription factor is reconstituted and initiates transcription from a target promoter. For construction of the light inducible split transcription factor, the DNA-binding domain TetR was fused to the N-terminal half of PIF6 (amino-acids 1-100) that was shown to be sufficient for selective binding to the P_{FR} form, but not to the P_{R} form of PhyB. The photosensory N-terminal domain of PhyB was fused to the transactivator-domain VP16. This red light responsive split transcription factor is expressed from a bicistronic expression unit (Fig. 1A) and directs reporter gene expression from a construct consisting of several repeats of the tetO-motif that tethers TetR-PIF6 and is linked to a minimal human cytomegalovirus immediate early promoter (P_{hCMVmin}). While the natural chromophore phytochromobilin is not found in mammalian cells, it can be substituted for phycocyanobilin (PCB) that is conveniently extracted from the algae *Spirulina* and is autoligated to PhyB upon addition to the culture medium. Upon illumination with red light, PhyB_{FR} interacts with PIF6 enabling VP16-induced expression of the reporter gene that is switched off by illumination with far-red light (Fig. 1 B).

In order to find the optimal configuration of the system, red light induced transgene expression was compared using two C-terminally truncated PhyB variants. Considering that the mechanism of PhyB import into the nucleus in plants is only beginning to emerge and no data is available for mammalian cells, it was also tested whether targeting the PhyB fusion proteins to the nucleus by including a simian virus 40 derived nuclear localization signal could improve the system. Moreover, the effect of an increasing copy number of the tet-operator sequence in the reporter construct was investigated and, considering that the PhyB fusion protein is rather bulky, whether increasing the distance between the tet-operator and the minimal promoter would have a positive effect on expression strength and induction ratio (Fig. 1A).

Using human placental secreted alkaline phosphatase (SEAP) in transiently transfected CHO-K1 cells as a reporter, it was found that PhyB(1-650)-VP16-NLS (Fig. 2A) co-expressed with TetR-PIF6(1-100) yielded the best expression strength and induction ratio from a reporter construct with 13 repeats of the tet-operator (Fig. 2B) that is positioned 356 bp upstream of the minimal promoter (Fig. 2C). In all further experiments, this optimized system was used, consisting of the light responsive split transcription factor PhyB(1-650)-VP16-NLS and TetR-PIF6(1-100), combined with the reporter construct tetO₁₃-356bp-*goi*-pA. With this configuration induction levels between 25- and 65-fold and relative SEAP activity of about 20 % as compared to SEAP activity from a constitutive tet-transactivator, TetR-VP16, with the same reporter construct were routinely achieved. It was found that the system of the present invention is functional in all mammalian cells lines tested that include cells from human, hamster, mouse and monkey, demonstrating its general applicability across species in mammals (Fig. 2D).

### Example 2:

### Reversible activation of transcription.

For gene expression to occur at the will of the researcher, gene expression should be inducible and reversible at any point in time. The reversibility of gene expression was tested with several cycles of varying irradiation wavelengths on transiently transfected CHO-K1 cells (pKM022 + pKM006) and it was found to be fully reversible in all the configurations tested (Fig. 3A). In order to assess the OFF kinetics of the system, the termination of transcription was compared by illumination with 740 nm to the tetracycline-induced termination that is known to result in an immediate dissociation of TetR from its operator. Since the shut-off follows the same kinetics at 740 nm when compared to tetracycline induced termination of transcription, it was concluded that illumination with 740 nm leads to an immediate dissociation of PhyB-VP16-NLS from the promoter site (Fig. 3B).

It is known that the P_{FR} form of PhyB is not only converted to P_{R} by far-red light but that this process also takes place in the dark with slower kinetics (dark reversion) and results in a reduction of P_{FR} by about 20% in transgenic yeast. Therefore, we examined whether dark reversion affects the light induced system of the present invention by illuminating transiently transfected CHO-K1 for varying periods of time followed by incubation in the dark. The respective data do not show a significant decrease in reporter production due to dark reversion (Fig. 3C).

### Example 3:

### Adjustable and space resolved reporter gene expression.

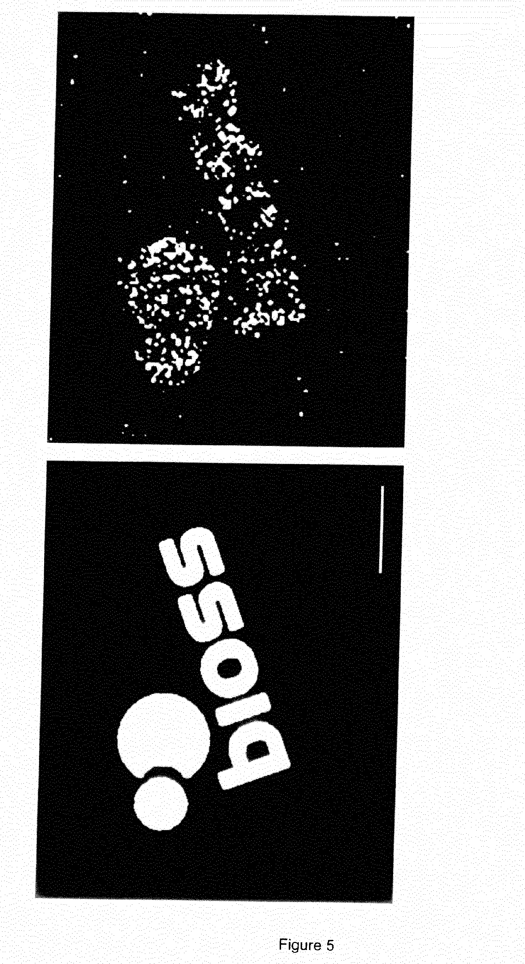

For biomedical applications it is important to titrate gene expression into the therapeutic window, and the expression of difficult-to-produce biopharmaceuticals that impair the viability of the production cell lines requires the precise control of production levels. Therefore, it was shown next that the system of the present invention can be employed for adjustable protein expression by either controlling the concentration of exogenously added chromophore (Fig. 4A) or by adjusting the light intensity (Fig. 4B). A major advantage of light over chemical inducers is the ability to spatially control gene expression. This was demonstrated by illuminating a monolayer of cultured cells transfected with a mcherry reporter and the light-inducible split transcription factor of the present invention with a defined pattern of red light, resulting in reporter gene expression in the illuminated areas only (Fig. 5).

### Example 4:

### Red light controlled vascularization in chicken embryos using conditional hVEGF₁₂₁ expression.

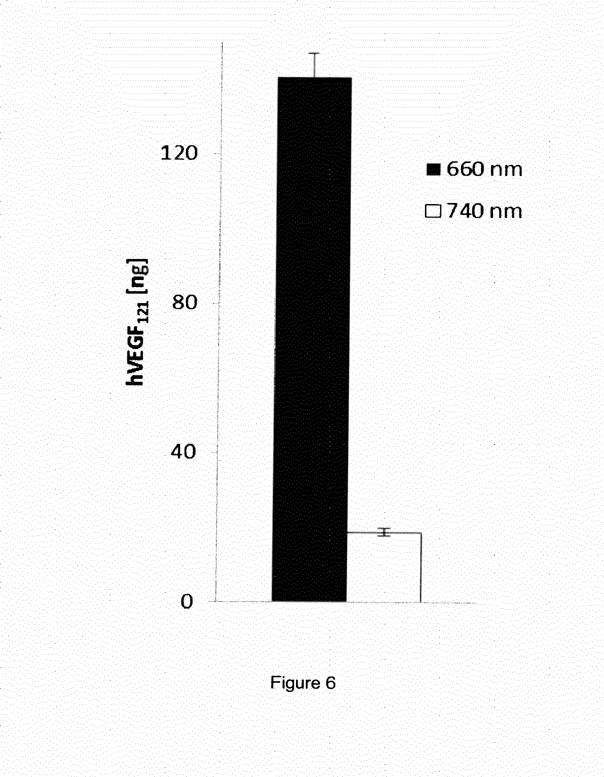

As a proof-of-concept that the red light inducible gene expression system of the present invention can be used for *in-vivo* cell-therapy, CHO-K1 cells engineered for red light inducible hVEGF₁₂₁ expression were placed onto chorioallantoic membranes (CAM) of developing chicken embryos that are a well-studied model for angiogenesis. For *in vivo* cell therapy, applied cells should ideally be contained at the side of application. In order to meet this requirement, transiently transfected (pKM022 + pKM033) CHO-K1 cells (Fig. 6) were immobilized in polyethylene glycol (PEG)-gels before application onto the CAMs of 10-day old chicken embryos. The CAMs were irradiated with red or far-red light for 48 hours, before microscopic analysis of vascularization.

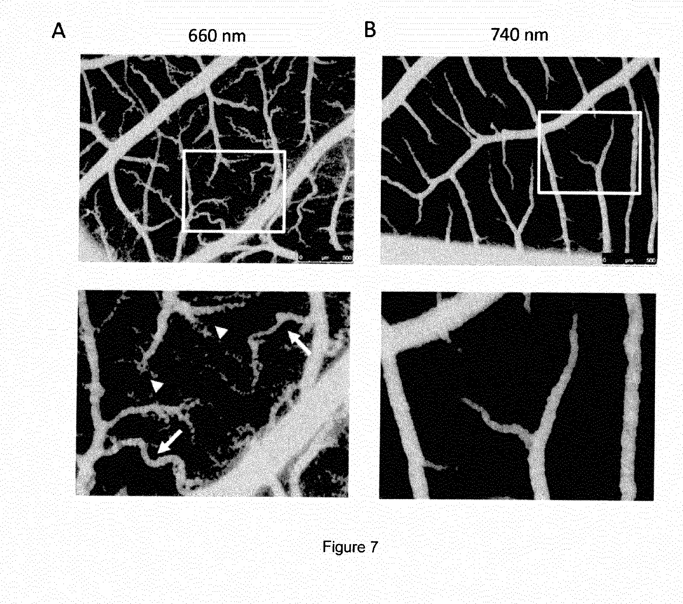

hVEGF₁₂₁-mediated vascularization could only be monitored in chicken embryos illuminated with 660 nm light, whereas control embryos illuminated at 740 nm showed a regular wild-type pattern of blood vessels (Fig. 7 and 8).

### Example 5:

### Comparison of the light-inducible transgene expression system of the present invention to US-Patent 6,887,688 B2.

In accordance with US-Patent 6,887,688 B2, PhyA from *Oryza sativa* or PhyB from *Arabidopsis thaliana* was fused to the DNA-binding domain TetR and the transactivation domain VP16 on pKM070 or pMZ320 respectively. The light inducible split-transcription factor of the present invention is produced from pKM022, pMZ324 codes for PIF6(1-100) with a nuclear localization sequence (NLS) and pKM006 is the SEAP reporter.

For the light-inducible system of the present invention pKM022 was co-transfected with pKM006. For the above patent pKM070 or pMZ320 was co-transfected with pKM006 and in additional experiments pMZ324 was added to these configuration. The experiments were conducted in CHO-K1 cells. 24 hours post transfection the culture medium was replaced and the cells were exposed to red or far-red light. Reporter gene assay was performed another 24 hours later.

The light-inducible expression system of the present invention showed manifold inducible expression of the reporter gene. In contrast, the systems in accordance with the above patent did not show any inducible transgene expression at all (Fig. 9).

## Claims

1. System for the light-regulated expression of a transgene in a mammalian cell, comprising:
(a) a nucleic acid comprising the coding sequence of a first fusion protein, the first fusion protein comprising:
(i) a photoreceptor protein;
(ii) a transcriptional activator or a DNA-binding protein; and
(iii) a nuclear localization sequence (NLS); and
(b) a nucleic acid comprising the coding sequence of a second fusion protein, the second fusion protein comprising:
(i) a DNA-binding protein or a transcriptional activator; and
(ii) a factor that interacts with the photoreceptor protein in a light-dependent manner;
wherein in case the first fusion protein comprises a transcriptional activator, the second fusion protein comprises a DNA-binding protein, and in case the first fusion protein comprises a DNA-binding protein, the second fusion protein comprises a transcriptional activator;
and further comprising:
(c) a nucleic acid comprising in 5' to 3' direction:
(i) at least one copy of an operator sequence which can be bound by the DNA-binding protein;
(ii) a promoter that is responsive to activation by the transcriptional activator; and
(iii) the coding sequence of the transgene under control of said
promoter;
**characterized in that** expression of the transgene can be reversibly activated by illumination with light of a suitable wavelength, and reversibly inactivated by illumination with light of a suitable wavelength.

2. The system of claim 1, wherein the nucleic acids (a) and (b) are present on one plasmid vector.

3. The system of claim 1 or claim 2, wherein the mammalian cell is selected from the group consisting of CHO cells (Chinese hamster ovary cells), CHO-K1 cells, NIH/3T3 cells, MEF cells (mouse embryonic fibroblast cells), COS-7 cells, HEK293 cells (human embryonic kidney cells), HEK293T cells, PER C6 cells, HUVEC cells, BHK-21 cells, HeLa cells, HT1080 cells, PC12 cells, C2C12 cells, Jurkat cells, K-562 cells, and human embryonic stem cells.

4. The system of any one of claims 1 to 3, wherein the photoreceptor protein is a phytochrome; preferably a phytochrome selected from the group consisting of Phytochrome A (PhyA), Phytochrome B (PhyB), Phytochrome C (PhyC), Phytochrome D (PhyD), and Phytochrome E (PhyE); more preferably PhyB; and most preferably the first 650 amino-terminal amino acids of PhyB.

5. The system of any one of claims 1 to 4, wherein the transcriptional activator is selected from the group consisting of acidic transcription activation domains, proline-rich transcription activation domains, serine/threonine-rich transcription activation domains, and glutamine-rich transcription activation domains; preferably wherein the transcriptional activator is selected from the group consisting of VP16, amino acid residues 753 to 881 of GAL4, the p65 domain of NF-κB, amino acid residues 399 to 499 of CTF/NF1, amino acid residues 31 to 76 of AP2, amino acid residues 1 to 427 of ITF1, amino acid residues 2 to 451 of ITF2, amino acid residues 175 to 269 of Oct1, and amino acid residues 132 to 243 of Sp1; more preferably wherein the transcriptional activator is VP16.

6. The system of any one of claims 1 to 5, wherein the DNA-binding protein is selected from the group consisting of members of the TetR family of transcriptional repressors, members of the Lacl family of transcriptional repressors, members of the LexA family of transcriptional repressors, members of the LasR family of transcriptional activators, members of the family of zinc-finger DNA binding domains, and members of the family of Transcription activator-like effector (TALE) DNA binding domains; preferably wherein the DNA-binding protein is TetR.

7. The system of any one of claims 1 to 6, wherein the factor that interacts with the photoreceptor protein in a light-dependent manner is selected from the group consisting of Phytochrome Interacting Factors (PIFs), FHY1/FHL, Phytochrome kinase substrate 1 (PKS1), nucleoside diphosphate kinase 2 (NDPK2), cryptochromes such as CRY1 and CRY2, Aux/IAA proteins, phosphatases such as FyPP and PAPP5, E3 ubiquitin ligases such as COP1, and ARR4; preferably wherein said factor is selected from the group consisting of PIF1, PIF2, PIF3, PIF4, PIF5, PIF6, PIF7; wherein said factor more preferably is PIF6; most preferably the first 100 amino-terminal amino acids of PIF6.

8. The system of any one of claims 1 to 7, wherein the operator sequence which can be bound by the DNA-binding protein is selected from the group consisting of the tet operator (tetO), the lac operator, and the lex operator; preferably wherein said operator sequence is tetO.

9. The system of any one of claims 1 to 8, wherein the promoter that is responsive to activation by the transcriptional activator is selected from the group consisting of the minimal human Cytomegalovirus immediate early promoter (p_{hCMVmin}), the minimal Drosophila HSP90 promoter, and the minimal Interleukin-2 promoter, preferably the minimal human Cytomegalovirus immediate early promoter (p_{hCMVmin}),.

10. The system of any one of claims 1 to 9, wherein the nucleic acid (c) comprises 1 to 100, preferably 5 to 30, more preferably 10 to 16, and most preferably 13 consecutive copies of the operator sequence.

11. The system of any one of claims 1 to 10, wherein nucleic acid (c) comprises a spacer region downstream of the operator sequence and upstream of the promoter, wherein the spacer has a length of 1 to 1000 basepairs (bp), preferably 250 to 600 bp, more preferably 320 to 390 bp, more preferably 350 to 360 bp, and most preferably 356 bp.

12. An isolated nucleic acid molecule comprising coding sequences of:
(a) a first fusion protein comprising:
(i) a photoreceptor protein;
(ii) a transcriptional activator or a DNA-binding protein; and
(iii) a nuclear localization sequence (NLS);
and
(b) a second fusion protein comprising:
(i) a DNA-binding protein or a transcriptional activator; and
(ii) a factor that interacts with the photoreceptor protein in a light-dependent manner;
wherein in case the first fusion protein comprises a transcriptional activator, the second fusion protein comprises a DNA-binding protein, and in case the first fusion protein comprises a DNA-binding protein, the second fusion protein comprises a transcriptional activator.

13. The isolated nucleic acid molecule of claim 12, wherein the photoreceptor protein is as defined in claim 4, and/or the transcriptional activator is as defined in claim 5, and/or the DNA-binding protein is as defined in claim 6, and/or the factor that interacts with the photoreceptor protein in a light-dependent manner is as defined in claim 7.

14. The isolated nucleic acid molecule of claim 12 or claim 13, further comprising an internal ribosome entry site (IRES) and/or a sequence coding for a 2A-peptide between the coding sequences for the first fusion protein and the second fusion protein.

15. A vector comprising the nucleic acid of any one of claims 12 to 14.

16. A mammalian host cell comprising at least one of the following elements:
(a) the expression system of any one of claims 1 to11;
(b) the isolated nucleic acid molecule of any one of claims 12 to 14; and
(c) the vector of claim 15.

17. A method for the light-regulated expression of a transgene in a cell, comprising the steps of:
(a) providing a cell comprising a nucleic acid, said nucleic acid comprising in 5' to 3' direction:
(i) at least one copy of an operator sequence which can be bound by a DNA-binding protein;
(ii) a promoter that is responsive to activation by a transcriptional activator; and
(iii) the coding sequence of the transgene under control of said promoter;
(b) expressing in said cell
a first recombinant fusion protein, comprising:
(i) a photoreceptor protein;
(ii) the transcriptional activator or the DNA-binding protein; and
(iii) a nuclear localization sequence (NLS);
and a second recombinant fusion protein, comprising:
(i) the DNA-binding protein or the transcriptional activator; and
(ii) a factor that interacts with the photoreceptor protein in a light-dependent manner;
wherein in case the first fusion protein comprises the transcriptional activator, the second fusion protein comprises the DNA-binding protein, and in case the first fusion protein comprises the DNA-binding protein, the second fusion protein comprises the transcriptional activator;
and
(c) subjecting said cell to light having a suitable wavelength, in order to activate transcription of the transgene.

18. The method of claim 17, wherein the cell is as defined in claim 3, and/or the photoreceptor protein is as defined in claim 4, and/or the transcriptional activator is as defined in claim 5, and/or the DNA-binding protein is as defined in claim 6, and/or the factor that interacts with the photoreceptor protein in a light-dependent manner is as defined in claim 7, and/or the operator sequence is as defined in claim 8, and/or the promoter that is responsive to activation by the transcriptional activator is as defined in claim 9, and/or the nucleic acid defined in step (a) is as defined in claim 10, and/or the nucleic acid defined in step (a) is as defined in claim 11.

19. A kit, comprising at least one of the following elements:
(a) the isolated nucleic acid as defined in any one of claims 12 to 14;
(b) the vector as defined in claim 15; and
(c) the mammalian host cell as defined in claim 16.

20. The kit of claim 19, further comprising an isolated nucleic acid comprising in 5' to 3' direction:
(a) at least one copy of an operator sequence which can be bound by a DNA-binding protein;
(b) a promoter that is responsive to activation by a transcriptional activator; and
(c) a cloning site for placing a transgene of interest under control of said promoter.

21. The kit of claim 20, wherein the operator sequence which can be bound by the DNA-binding protein is as defined in claim 8, and/or the promoter that is responsive to activation by the transcriptional activator is as defined in claim 9, and/or the nucleic acid is as defined in claim 10, and/or the nucleic acid is as defined in claim 11.

22. The expression system of any one of claims 1 to 11, the isolated nucleic acid of any one of claims 12 to 14, the vector of claim 15, the mammalian host cell of claim 16, or the kit of any one of claims 19 to 21, for use in medicine, preferably for use in tissue engineering, skin regeneration, tissue regeneration, expression of therapeutic proteins, or induction of blood vessel formation.
